# EUROPEAN PATENT APPLICATION

(11) **EP 2 706 358 A2**
(43) Date of publication of application: **12.03.2014**
(21) Application number: 13004904.2
(22) Date of filing: 14.12.2009
(51) Int. Cl.: G01N 33/574

(54) **Biomarkers for inhibitors with anti-angiogenic activity**

(30) Priority: 23.12.2008 EP 08022436
(62) Divisional of application: 09775114.3
(71) Applicant: Merck Patent GmbH, 64293 Darmstadt (DE)
(72) Inventor: Behrens, Joyce, 81667 München (DE)

(57) **Abstract**

The invention relates to a method for assessing the effect of the integrin inhibitors and small molecule ATP site directed multi kinase inhibitors DI-17E6 (abituzumab), cilengitide, sutinib and sorafenib on angiogenesis associated diseases by the use of certain identified biomarkers. The invention further relates to the use of these drugs for optimizing and scheduling the efficacy of the therapy.

## Description

### Field of the Invention

The invention relates to a method for assessing the effect of integrin inhibitors and small molecule ATP site directed multi-kinase inhibitors like DI-17E6 (abituzumab), cilengitide, sutinib and sorafenib on angiogenesis by the use of certain identified biomarkers. This method is notably beneficial for the determination of the efficacy of integrin inhibitors and small molecule ATP site directed multi-kinase inhibitors mainly used for the treatment of angiogenesis associated diseases such as cancer. Especially, the invention relates to biomarkers linked to angiogenesis that are preferably accessible in body fluids and therefore allow analysis of target modulation in a non-invasive way. The invention further relates to the use of these drugs for optimizing and scheduling the efficacy of the therapy.

### Background of the invention

Angiogenesis, is generally applied to the formation of new blood vessels from preexisting vasculature. The proliferation of endothelial cells undergoing DNA synthesis is a common hallmark of angiogenic microvascular vascular sprouts whereas extensive sprouts mainly require the migration of endothelial cells. Angiogenesis is a fundamental process that is orchestrated by a range of angiogenic factors and inhibitors. Activators of endothelial cell proliferation and migration are mainly receptor tyrosine kinase ligands, such as vascular endothelial growth factor (VEGF), fibroblast growth factors (FGFs), plateled derived growth factor (PDGF) and epidermal growth factor (EGF), but can also be of very different origin, such as lysophosphatic origin (LPA) (Bergers et al. (2003) Nature Reviews 3, p.401-410). EGF upregulates VEGF, FGF and interleukin-8 (IL-8), whereas LPA upregulates VEGF levels. The first described angiogenic inhibitor was thrombospondin-1, which modulates endothelial cell proliferation and motility. Remarkably, many inhibitory molecules, such as statins, are derived from larger proteins that have no effect on angiogenesis; e.g. Angiostatin, a fragment of plasminogen that binds ATP synthase and annexin III as well as endostatin, tumstatin and canstatin (fragments of collagens that bind to integrins). In general, the levels of activators and inhibitors dictate whether an endothelial cell will be in a quiescent or an angiogenic state. It is believed that changes in the angiogenic balance mediate the angiogenic switch.

Its physiological role lies in the development, reproduction and repair of cells, tissues and organs. Pathological angiogenesis is necessary for tumors and their metastasis to grow beyond a microscopic size and it can give rise to bleeding, vasculare leakage and tissue destruction. The consequences of pathological angiogenesis can be responsible, directly or indirectly, for the symptoms, incapacitation or death associated with a broad range of angiogenesis associated disease. Examples of such disease include tumor disorders, autoimmune diseases, macular degeneration and atheriosclerosis among others.

Regarding tumor progression the angiogenic switch can occur at different stages on the tumor progression pathway, depending of the tumor type and the environment. However, neovascularization is usually a prerequisite of tumor progression.

Several signalling pathway are involved in the process of angiogenesis. VEGF plays a pivotal role in vasculogenesis and angiogenesis; of all the angiogenic factors, VEGF is the most frequently associated with tumor progression and metastasis. Preliminary evidence suggests that overexpression of various isoforms may have differential effects on tumor.

Key steps in the tumor-associated angiogenesis includes the secretion of VEGF by tumor cells which binds to its receptor VEGFR2 and to neuropilin on endothelial cells (Folkman 2007, Nature Reviews, 6, p.273-286). It's the most common of at least six other pro-angiogenic factors from tumors. Matrix metalloproteases (MMPs) are released from tumor cells, but also by VEGF-stimulated cells. MMPs mobilize pro-angiogenic proteins from stroma, but also cleave the angiogenic inhibitor endostatin from collagen 18 in the vessel wall and participate in the cleavage of the angiogenic inhibitor angiostatin from circulating plasminogen. Tumor cells secrete angiopoetin 2 (ANGPT2) which competes with ANGPT1 for binding to the endothelial TIE2 receptor. Angiopoietin 1 (ANGPT1), expressed by many cells, binds to the endothelial TIE2 (also known as TEK) receptor and helps maintain a normalized state in blood vessel. ANGPT2 increases the degradation of vascular basement membrane and migration of endothelial cells, therefore facilitating sprout formation. PDGF, secreted by many tumors, can upregulate its own receptor (PDGFR) on endothelial cells. Basis fibroblast growth factor (bFGF) is secreted by other tumors. In response to growth regulators such as VEGF and bFGF endothelial cells upregulate certain integrins. Integrins are cell surface adhesion molecules which facilitate endothelial binding to extracellular enviroment, a requirement for cells to maintain viability and responsiveness to growth regulatory proteins. Endothelial cells are the most anchorage-dependent cells. Integrins are thought to sustain endothelial cell viability during the intermittent detachments that are required to migrate towards a tumor and to increase their sensivity to growth regulators such as VEGF and bFGF.

Integrins plays a key role in tumor angiogenesis and metastasis, and several other angiogenesis-dependent human disease like psoriasis and rheumatoid arthritis (Mousa SA, Drugs of the Future 1998; 23(I):51-60). They are cell surface adhesion molecules coupling the extracellular environment to the cytoskeleton (Geiger B. et al., Nat Rev Mol Cell Biol 2001; 2:793-805). In addition to their structural functions, they are receptors for transmitting signals important for cell adhesion, migration, proliferation and survival.

Signalling events induced by integrins are multiple and depend on the cell context. Ligand binding to the extracellular integrin domain induces conformational changes and integrin clustering leading to activation of signalling cascades and recruitment of multiprotein complexes to focal adhesions (Geiger B. et al., Nat Rev Mol Cell Biol 2001; 2:793-805). Messages are transmitted through a variety of intracellular protein kinases and adaptor molecules including Ras, MAP kinase (MAPK), focal adhesion kinase (FAK), Src, Rac/Rho/cdc42 GTPases, PKC and PI3K. Via these molecules, integrin signaling tightly and cooperatively interacts with receptor tyrosine kinase signaling to regulate survival, proliferation, adhesion and migration (Giancotti FG. et al., Science 1999; 285(5430):1028-32).

Integrins form heterodimers consisting of two subunits, alpha and beta (Hynes RO. Trends Cell Biol 1999; 12: 33-37). They are differentially expressed on various cell types and recognize multiple ligands of the extracellular matrix proteins like collagen, vitronectin, fibronectin and laminin. A subset of integrins including alpha(V)beta(3) and alpha(V)beta(5) recognize a common motif in their ligands, the RGD sequence, that is present in ligands such as vitronectin, fibronectin, fibrinogen (Ruoslahti E. Ann. Rev. Cell Dev. Biol. 1996. 12: 697-715).

αvβ5 integrin specifically binds vitronectin, while αvβ3 also binds other macromolecules of the provisional ECM. αvβ3 binds e.g. ligands including fibrin, fibrinogen, laminin, thrombospondin, vitronectin, von Willebrand's factor, osteospontin and bone sialoprotein I. The specific cell adhesion roles of these integrins play in the many cellular interactions in tissues is still under investigation, but it is clear that there are different integrins with different biological functions.

αvβ3 is highly expressed on activated endothelial cells, such as tumor endothelial cells, but not on resting endothelial cells and most normal organ systems, which makes it a suitable target for anti-angiogenic therapy (Brooks PC et al., Science 1994; 264:569-71). αvβ3 was first noted in cancer as a progression-dependent marker on malignant melanoma. It enhanced melanoma growth in vivo and survival in vitro. αvβ3 blockers reversed these effects. Subsequently, αvβ3 was found in other tumors including glioblastoma, renal carcinomas, ovarian carcinomas and others. αvβ3 was also widely over-expressed in the ECs in many malignancies. In vitro, angiogenic models activated by tumor-derived growth factors over-expressed and required αvβ3 on the sprouting vasculature, while αvβ3 and αvβ5 blockade could suppress the angiogenic phenotype. αvβ5 was also shown to support the neo-vasculature induced by some tumor-derived growth factors. Inhibiting αvβ5 can trigger apoptosis of tumor cells.

Integrin inhibitors have been suggested as pharmaceutically active principle in human and veterinary medicine, in particular for the prophylaxis and treatment of mainly angiogenesis associated disorders. They can be used for the treatment and prophylaxis of the circulation, thrombosis, cardiac infarction, arteriosclerosis, inflammations, apoplexy, angina pectoris, tumor disorders, osteolytic disorders, especially osteoporosis, angiogenesis and disorders resulting from angiogenesis for example diabetic retinopathy of the eye, macular degeneration, myopia, ocular histoplasmosis, rheumatic arthritis, osteoarthritis, rubeotic glaucoma, and also ulcerative colitis, Crohn's disease, multiple sclerosis, psoriasis and restenosis following angioplasty.

There are several integrin inhibitors in clinical development for the treatment of cancer, arthritis and psoriasis, such as cilengitide, abegrin (vitaxin, MEDI-522), CNT095, DI-17E6 and volociximab (Nemeth JA et al., Cancer Invest. 2007; 25(7): 632-646). They antagonize integrin interaction with the extracellular matrix (ECM), and perturbs the function of endothelial and tumor cells. Said integrin inhibitors are characterised by distinct specificities and mechanism of actions. Abegrin, volociximab, DI-17E6 and CNT095 are monoclonal antibody antagonists.

Cilengitide is an antagonistic cyclic peptide (cyclo-(Arg-Gly-Asp-DPhe-NMeVal)) mimicking the RGD-motif (EP 0770622). Cilengitide targets the αv- integrins, in particular αvβ3 and αvβ5.

US6521593 discloses methods for the inhibition of tumor growth in brain, using cilengitide as αᵥβ₃/αᵥβ₅ integrin inhibitors. The invention includes that said integrin inhibitors show an anti-tumorgenic effect which depends of the anti-angiogenesis. Additionally said integrin antagonism may induce direct tumor cell death which is independent of anti-angiogenesis. WO02/41910 relates to the use of cilengitide as αᵥβ₃/αᵥβ₅ integrin inhibitors for the treatment of diseases of the eye of the patient resulting from the angiogenesis.

Monoclonal mouse antibody 17E6 inhibits specifically the αv- integrin subunit of human integrin receptor bearing cells. The mouse IgG1 antibody is described, for example by Mitjans et al. (1995; J.Cell Sci. 108, 2825) and patents US 5,985,278 and EP 719 859. Murine 17E6 was generated from mice immunized with purified and Sepharose-immobilized human αvß3. Spleen lymphocytes from immunized mice were fused with murine myeloma cells and one of the resulting hybridoma clones produced monoclonal antibody 17E6. DI-17E6 (CAS No: 1105038-73-0, abituzumab) is an antibody having the biological characteristics of the monoclonal mouse antibody 17E6 but with improved properties above all with respect to immunogenicity in humans. The complete variable and constant amino acid sequence of this modified antibody is presented in Figure 1 and in WO 2009/010290.

Tyrosine kinase inhibitors e.g. against VEGF and VEGFR have been developed with the scope to use them for anti-angiogenic therapies. The tyrosine kinase inhibitors of VEGFRs are low-molecular weight, ATP-mimetic proteins that bind to the ATP-binding catalytic site of the tyrosine kinase domain of VEGFRs resulting in blockage of intracellular signaling (Morabito et al 2006 The Oncologist 11: 753-764). Several of these agents are currently in different phases of clinical development. Large randomized phase II trials have demonstrated the efficacy of sunitinib and sorafenib in the treatment of patients affected by gastrointestinal stromal tumors (GISTs) and renal cancer, refractory to standard therapies, respectively.

Sunitinib (SU011248, sutent) is an oral small molecular tyrosine kinase inhibitor that exhibits potent antiangiogenic and antitumor activity (O' Farrell AM et al., 2003. Blood 101: 3597-3605). It is a small molecule ATP site directed competitive inhibitor. Tyrosine kinase inhibitors such as SU6668 and SU5416 (semaxanib) demonstrated poor pharmacologic properties and limited efficacy; therefore, sunitinib was rationally designed and chosen for its high bioavailability and its nanomolar-range potency against the antiangiogenic receptor tyrosine kinases (RTKs)-vascular endothelial growth factor receptor (VEGFR) and platelet-derived growth factor receptor (PDGFR). Sunitinib inhibits other tyrosine kinases including, *KIT, FLT3,* colony-stimulating factor 1 (CSF-1), and *RET,* which are involved in a number of malignancies including small-cell lung cancer, GI stromal tumors (GISTs), breast cancer, acute myelogenous leukemia, multiple endocrine neoplasia types 2A and 2B, and familial medullary thyroid carcinoma. Sunitinib demonstrated robust antitumor activity in preclinical studies resulting not only in tumor growth inhibition, but tumor regression in models of colon cancer, non-small-cell lung cancer, melanoma, renal carcinoma, and squamous cell carcinoma, which were associated with inhibition of VEGFR and PDGFR phosphorylation. Clinical activity was demonstrated in neuroendocrine, colon, and breast cancers in phase II studies, whereas definitive efficacy has been demonstrated in advanced renal cell carcinoma and in imatinib-refractory GISTs, leading to US Food and Drug Administration approval of sunitinib for treatment of these two diseases. Studies investigating sunitinib alone in various tumor types and in combination with chemotherapy are ongoing. The clinical benchmarking of this small-molecule inhibitor of members of the split-kinase domain family of RTKs will lead to additional insights regarding the biology, potential biomarkers, and clinical utility of agents that target multiple signaling pathways in tumor, stromal, and endothelial compartments.

Sorafenib is a novel bi-aryl urea, initially developed as a specific inhibitor of Raf kinase signaling (Morabito et al 2006 The Oncologist 11: 753-764). Subsequent studies have shown this compound to also inhibit several other tyrosine kinases involved in tumor progression, including VEGFRs. Xenograft models of colon, breast, and non-small cell lung cancer (NSCLC) treated with sorafenib demonstrated significant inhibition of tumor angiogenesis, as measured by anti-CD31 immunostaining. A large phase II randomized trial was conducted with sorafenib, in patients with different types of tumors. Results have been reported for 202 patients with advanced renal cell carcinoma. A subsequent randomized, placebo-controlled, phase III trial confirmed the efficacy of sorafenib in cytokine-refractory advanced renal carcinoma patients. Sorafenib was well tolerated, with manageable site effects: rash (34%), diarrhea (33%), hand-foot skin reactions (27%), fatigue (26%), and hypertension (11 %). Based on these results, the FDA announced in December 2005 the approval of sorafenib for patients with advanced renal cancer. Moreover, the European Commission recently granted orphan medicinal product status to sorafenib for the treatment of hepatocellular carcinoma. In a phase II trial, 43% of patients treated with sorafenib experienced stable disease for at least 4 months and an additional 9% of patients experienced tumor shrinkage. Currently, phase III clinical trials are evaluating the efficacy of sorafenib in hepatocellular carcinoma, metastatic melanoma, and NSCLC; moreover, several other phase I/II studies are ongoing with sorafenib combined with several chemotherapeutic (irinotecan, dacarbazine) or molecular targeted (gefitinib) agents in advanced solid tumors, to maximize the therapeutic potential of the drug.

Anti-angiogenic therapy primarly targets the activated vascular endothelial cells e.g. in a tumor. It can accomplish this directly by preventing endothelial cells from responding to the environment via inhibition of integrins as described above. Anti-angiogenic therapies can also acting by neutralization of angiogenic factors as bevacizumab does (see below).

Genetic variations of e.g. angiogenic factors have important consequences for the clinical treatment of angiogenesis-associated diseases. For example, individuals with Down syndrome have a 1.6 higher fold level of endostatin than normal individual because of an extra copy of the endostatin precursor gene. They seem to be most protected from all humans against cancer. Therefore, differences in angiogenesis due to genetic variations should be considered in anti-angiogenic therapies.

Anti-angiogenic therapy might optimally require that endothelial cells be exposed to steady state blood levels of the inhibitors. Therefore, often dosing might be optimal for cyclopolypeptide inhibitors such as cilengitide. However, monoclonal antibodies such as D7E16 might be administered in larger intervals because of long lasting antibodies.

Tumors might became refractory to anti-angiogenic therapy, especially if a mono-anti-angiogenic therapy targets only one angiogenic protein (for example VEGF). With anti-VEGF-therapy (such as an antibody to VEGF (bevacizumab), the vasculature of early tumors decreases and the tumor growth is limited. However, in retrospective analyses a continued anti-angiogenic treatment of metastatic tumors with bevacizumab did not result in a benefit. Although VEGF is expressed by up to 60% of human tumors, most tumors can also express five to eight other angiogenic proteins. When the expression is suppressed for a long time, the expression of another angiogenic protein might emerge (Dorrell et al (2007) Proc. Natl. Acad. Sci doi:10.1073/ pnas.0607542104).

Therefore, for the above described reasons a panel of sensitive and specific biomarkers elucidating linked signal pathways could assist in the choice of anti-angiogenic therapies. Furthermore, they might support the diagnosis of a microscopic recurrent tumor even before it could be anatomically located.

Besides, tumors poorly vascularized will be inhibited by a significantly lower dose of an angiogenesis inhibitor than is required for a highly vascularized tumor. These tumors might express their own angiogenesis inhibitors and might respond to a lower dose of an angiogenesis inhibitor.

Genetic variations and diverse angiogenic processes e.g. in the broad range of tumor types and their development require inter alia a broad panel of informative biomarkers to optimize and to individualize an anti-angiogenic therapy.

It might also be possible to use angiogenesis-based biomarkers to monitor the progression or regression of certain angiogenesis-dependent disease that are non-neoplastic. These include e.g. atherosclerosis, endometriosis, Crohn's disease and rheumatoid arthritis, among others.

### SUMMARY OF THE INVENTION

The present invention bases on the various experiences that anti-angiogenic therapeutics require informative molecular biomarkers (Bertolini F et al. Drug Discov Today. 2007; 12(19-20):806-12). Said biomarkers could preferably be used e.g.
(1) for the measurement of the molecular target status and pharmacodynamic (PD) endpoints of drug effects on target,
(2) for the identification of angiogenesis linked pathways
(3) to find the most appropriate anti-angiogenic drugs for individual patients
(4) to provide proof of concept for target modulation
(5) to test underlying hypotheses
(6) provide rational selection of dose and schedule of an anti-angiogenic therapy
(7) to explain or predict clinical outcomes.

Despite many successes of molecular cancer therapeutics such as trastuzumab and imatinib, only few oncology drugs that enter the clinic make it to marketing approval. The use of molecular biomarkers should reduce this high level of attrition, thereby reducing costs of drug development and increasing the likelihood of getting innovative and active drugs to cancer patients.

These molecular biomarkers could complement the information which is given by cellular biomarkers. Cellular biomarkers are e.g. different endothelial cell populations such as circulating endothelial cells and circulating endothelial progenitors that are used as marker for angiogenesis. Molecular biomarker could compensate the disadvantages of cellular biomarkers such as subjective enumerations of endothelial cell populations. The use of said cellular biomarkers requires much more work in the characterization of cancer-related endothelial (Bertolini et al (2007) Drug discovery today 12, 806-812). Also imaging techniques such as dynamic contrast magnetic resonance imaging (DCE-MRI) as a approach for the measurement of angiogenesis has some strong limits for a routine application. These techniques are expensive and restricted to centers with adequate instrumentation.

The present invention concerns methods of assessing the effect, efficacy, safety, prognosis, and / or dosing of therapeutic agents or drugs which are angiogenesis inhibitors, preferably integrin inhibitors or small molecule ATP site directed multi-kinase inhibitors for the treatment of patients suffering from angiogenesis related diseases, preferably cancer.

The present invention is further related to methods of assessing the effect of angiogenesis inhibitors preferably integrin inhibitors or small molecule ATP site directed multi-kinase inhibitors on angiogenesis as such.

The present invention also concerns methods for screening for integrin modulating compounds in a cell based model.

In a first aspect, the invention is directed to a method for assessing the effect of an angiogenesis inhibitor, preferably integrin or ATP site directed multikinase inhibitors on angiogenesis by means of specific informative molecular biomarkers. Thereby, the invention is based particularly on the need of assessing the efficacy of angiogenesis inhibitors that should applied to an individual preferably in the context of an anti-angiogenic therapy. Said anti-angiogenic therapy will be applied in order to treat angiogenesis associated disease that are described in the background section. In another aspect, the invention is directed to the use of said informative molecular biomarkers for determining and predicting the efficacy of such an inhibitor in the diagnosis and therapy of a patient suffering from an angiogenesis, preferably integrin related disease.

There are different hints that have to be considered in the establishment of informative molecular biomarkers which should assess the efficacy of angiogenesis inhibitors administered to an individual in the context of an anti-angiogenic therapy.

For instance, since anti-angiogenic therapies might be administered to individuals suffering from different kinds of angiogenesis-associated disease and different kinds of tissues and/ or organs are concerned by said disease it is difficult or for medical reasons even not possible to obtain sufficient biopsies for the analysis of molecular biomarkers. Moreover, if angiogenesis inhibitors are used e.g. to target a metastasing tumor in an individual different biopsies of various target tissues would be necessary in order to analyze the effect of said inhibitors. Therefore, the evaluation of angiogenesis dependent tumor progression due to the treatment with angiogenesis inhibitors would require certain different tissue samples.

Furthermore, since angiogenesis relies on the formation of new blood vessels and a lot of angiogenic steps involve proteins in circulation the success of an anti-angiogenic therapy cannot be evaluated by the analysis of the appearance or disappearance of molecular biomarkers in a certain tissue.

Hence, the use of circulating proteins as biomarkers might give information about the success of an anti-angiogenic therapy. Said circulating proteins might be detectable in body fluids and therefore they could be advantageous to overcome the discussed hints. Single chosen angiogenic growth factors such as VEGF, bFGF and PLGF have been analyzed in the blood plasma after the administration of certain anti-angiogenic therapies (Jubb et al. 2006, Nature Reviews, 6, 626-635). But the meaningful evaluation of angiogenic growth factors in the blood plasma is restricted due to the active uptake of many growth factors from platelets and other cells. Furthermore, tumor cells express distinct levels of said angiogenic growth factors. Besides, due to the complexity of angiogenesis and the complexity of protein circulation the evaluation of few single biomarkers in a sample is not efficient to evaluate anti-angiogenic therapies. Therefore, a panel of certain molecular biomarkers which preferably belong to different protein families and which are involved in a different manner to angiogenesis or associated signaling pathways might complement the lack of sufficient tools in the monitoring of anti-angiogenic drugs.

The invention relates to certain molecular biomarkers that are preferably secreted from tissue cells into their environment due to the administration of angiogenesis inhibitors to an individual and due to their binding to its selective target. Preferably, these molecular biomarkers might be secreted from tissue cells into their environment and as a consequence thereof, they might be detectable in body fluids. Hence, body fluids from an individual treated by said angiogenesis inhibitors were preferably used for the analysis of the molecular biomarkers.

Biomarkers which were found according to the present invention to be responsive to angiogenesis related receptors, preferably integrins an ATP site directed multi-kinases and inhibitors of theses receptors are the following ones:
ADAMTS1, ADAMTS9, SERPINB2, SERPIND2, STC1, ADAM15, SRPX, CORO1C, PBEF1, LTB4DH, IL15RA, ENPP4, PLAUR, LAMA5, TNFRSF1A, CLU, TOR3A, IL1RL1, IL1R1, TCN2, VEGFC, LMNA, CTSL, PTX3, NCL, GUSB, CLEC3B, IL8, GPX3, CXCL1, SDFR1, STAT1, CDCA7, DAF, HYAL2, CD40, NOTCH1, INHBA, EDN1, MCP1, MMP10, AUTS2, LIPG, PDGFB, BMP4, MGP, HTRA1, FBLN5, HHIP, COL4A1, GALNT, MMP2, IL13RA1, MFAP2, NTN4, DKK3. SERPINE2, COL5A1, CHST1, PGF, EFNA4, BCHE, ENG, ROR1, ANGPTL4, ESM1, CUBN, IGFBP7, CYR61, FLT4, SPOCK, COL12A1, LTBP1, MMP28, TMEM4, CTSB, COL1A2, GRP124, SPARC, FST, DKK4, PHLDB2, PVR, CYR61, MERTK, FSTL3, APLN, PTX3, CTGF, NRG1, DKK3, CD44, BCHE, BDNF, FBXO4, EFEMP1, STC2, IL-6, CRP, CD40 ligand and/or MMP9.

Biomarkers which are preferred according to the invention with respect to their responsiveness to the treatment of some specific angiogenesis inhibitors, are:
ADAMTS1, STC1, EDN1, MCP1, IL8, PDGF-BB, CRP, CD40 ligand, and IL6.

Most preferred biomarkers according to the present invention are ADAMTS1, STC1, EDN1, and IL8, or combinations thereof.

In a preferred embodiment, the molecular biomarkers can be detected in body fluids such as blood and blood plasma, lymph, liquor and/or urine. In a notably preferred embodiment, the molecular biomarker can be detected in blood and/or urine. In this case the biomarkers can be investigated easily, since blood and urine can be taken in a non-invasive way.

In a preferred embodiment of this invention, the angiogenesis inhibitor is selected from integrin inhibitors. In a more preferred embodiment, said integrin inhibitor selected from alpha (v) integrin inhibitors.

In a notably preferred embodiment, said integrin inhibitor is selected from DI-17E6 or its derivative and/or cilengitide (cyclo-(Arg-Gly-Asp-DPhe-NMeVal)). In doing so, the molecular biomarkers will be analyzed in body fluids obtained from an individual who has been treated with a assumingly therapeutically effective amount of a DI-17E6 or its derivative and/or cilengitide.

In another preferred embodiment, said integrin inhibitors bind specifically to integrins on endothelial cells. Preferably, the molecular biomarkers will be upregulated in said endothelial cells and get secreted to a higher amount into the environment of the endothelial cells. In another case, the secretion of said biomarkers is getting started due to the treatment with said inhibitors. The environment of endothelial cells comprises the lumen of the blood vessel and the neighboured tissue. In a notably preferred embodiment, the molecular biomarkers reach the blood stream after its secretion from the endothelial cells.

In another preferred embodiment of the invention, due to the specific binding of said integrin inhibitors to endothelial cells the molecular biomarkers are downregulated in said endothelial cells and get secreted to a lower amount into the environment of the endothelial cells. In another case, the secretion of said biomarkers is getting stopped due to the treatment with said inhibitors.

In one embodiment of this invention, said molecular biomarkers which can be detected in certain body fluids due to the treatment with integrin inhibitors are proteins. Preferably, said proteins are soluble proteins of the blood serum or proteins comprised in the blood plasma. In one embodiment of this invention, said proteins can be detected mainly by immunological based techniques such as Western Blot, Elisa, Luminex and related techniques.

For the purpose of the present invention, said biomarkers preferably proteins are either elevated or diminished quantitatively in the preferred body fluid in response to the treatment via integrin inhibitors. In a preferred embodiment, the elevation or diminishment of a certain biomarker due to the treatment of integrin inhibitors characterized in multiple trials is defined by a p-value not overrunning 0.01.

In another aspect of the present invention, said biomarkers preferably proteins are modified in response to the treatment with integrin inhibitors. These alterations might include a change in the molecular size of the protein, modifications such as glycosilations in the protein and interactions with different or same protein subunits.

In another aspect of this invention, the altered gene expression of said biomarkers can be detected on the RNA level. Thereby, RNA will be extracted from samples of tissue cells obtained from an individual in response to the treatment via integrin inhibitors. The expressed RNA transcripts of certain biomarkers can thus be analyzed by known gene expression profiling techniques such as array technology. Preferably, the samples of tissue cells which are taken for the RNA extraction derive from endothelial cells.

Preferably, the method for assessing the efficacy of integrin inhibitors in anti-angiogenic therapies comprises first the administration of an assumingly therapeutic effective amount of an integrin inhibitor to an individual. The assumption and determination of a therapeutically effective amount is based e.g. on previous preclinical and clinical research. Second, the efficacy of the administration of an assumingly therapeutic effective amount of said integrin inhibitor is assessed by the methods of said invention. The evaluation of said assessment is adducted to repeat the angiogenic therapy for further rounds under altered conditions.

In accordance with one embodiment of the present invention, the biomarkers are preferably candidates that have been associated with angiogenesis and might be involved in signal pathways that are linked to the integrin receptors. More preferably, the biomarkers might be involved in blocking angiogenesis following the administration of integrin inhibitors. Another aspect concerns the diversity of the group of biomarkers of said invention. Preferably, they cannot be grouped due to high sequence homology in one protein family and cannot be grouped due to their physiological relevance in an functional group such as the angiogenic factors VEGF, EGF and PLGF etc.

In another aspect of this invention, in order to assess an anti-angiogenic drug effect, biomarkers can be used separately, in combination with different biomarkers of said invention or with other angiogenesis biomarkers determined outside of this invention.

Additionally, the results of molecular biomarkers evaluation obtained by the determination of the protein levels in samples such as body fluids can be accomplished by the gene expression results obtained by the determination of the RNA levels in samples such as endothelial cells. Said comparison can be useful since the molecular biomarker in form of the protein transcript might e.g. not be stable in the blood or urine due to certain physiological circumstances.

In a second aspect of the present invention, the invention relates to a method for assessing the effect of an angiogenesis inhibitor by molecular biomarkers whereby the method comprises the following steps: A tissue sample was obtained from an individual before the administration of angiogenesis inhibitors wherein from the taken tissue sample cells preferably endothelial cells were prepared. Said cells were cultured under defined laboratory conditions *in vitro* and later on treated by angiogenesis inhibitors. Due to the treatment with angiogenesis inhibitors the expression and the secretion of the biomarkers might be changed. Said changed secretion can be followed in the cell culture medium. In detail, these molecular biomarkers are secreted from said cells into their environment i.e. the culture medium due to the treatment with angiogenesis inhibitors. As a result they are detectable in the cell culture medium. In this case, the molecular biomarkers can be investigated and monitored easily, since the cell culture medium of cultivated cells is easy to get and to handle.

In a preferred embodiment, the molecular biomarkers will be upregulated in the endothelial cells and get secreted to a higher amount into the culture medium of the endothelial cells. In another case, the secretion of said biomarkers is getting started due to the treatment with said inhibitors. In another preferred embodiment of the invention, the molecular biomarkers are downregulated in the endothelial cells and get secreted to a lower amount into the culture medium of the endothelial cells. In another case, the secretion of said biomarkers is getting stopped due to the treatment with said inhibitors.

The method characterized by said steps comprises an angiogenesis inhibitor which is selected preferably from integrin inhibitors. In a more preferred embodiment, said integrin inhibitor is selected from alpha (V) integrin inhibitors.

In a notably preferred embodiment, said integrin inhibitor is selected from DI-17E6 or its derivative and/or cilengitide. In doing so, the molecular biomarkers will be analyzed in the cell culture medium obtained from endothelial cells treated with an assumingly effective amount of a DI-17E6 or its derivative and/or cilengitide.

In one embodiment of this invention, said biomarkers which can be detected in supernatants due to the treatment via integrin inhibitors are proteins as described in the foregoing sections.

For the purpose of the present invention said biomarkers preferably proteins are either elevated or diminuished quantitatively in the supernatant of endothelial cells sample in response to the treatment via integrin inhibitors. In a preferred embodiment, the elevation or diminishment of a certain biomarker due to the treatment of integrin inhibitors characterized in certain trials is defined by a p-value not overrunning 0.01.

In another aspect of the present invention said molecular biomarkers preferably proteins which are detected in the cell culture medium are modified as described in the foregoing sections.

In another aspect of this invention the change of the gene expression of said biomarkers can be detected on the RNA level by the extraction of the RNA from endothelial cells due to the treatment via integrin inhibitors. The expressed RNA transcripts of certain biomarkers can thus be analyzed by known gene expression profiling techniques such as array technology.

The method for assessing the effect of integrin inhibitors on angiogenesis in said cell culture system described in the above section comprises first the administration of an assumingly therapeutic effective amount of an integrin inhibitor to endothelial cells. The determination of an assumingly therapeutic effective amount is based e.g. on previous preclinical and clinical research. Second, the effect of the administration of an assumingly therapeutic effective amount of said integrin inhibitor is analysed by the molecular biomarker described in said invention. The evaluation of said assessment is adducted for the determination or the repetition of an anti-angiogenic therapy.

In another aspect, the method for assessing the effect of integrin inhibitors on angiogenesis in said cell culture system described in the above section comprises first the administration of an explorative amount of an integrin inhibitor to endothelial cells. The determination of an explorative amount is based e.g. on previous preclinical and clinical research. Second, the effect of the administration of an explorative effective amount of said integrin inhibitor is analysed by the molecular biomarker described in said invention. The evaluation of said analysis is adducted for further preclinical or clinical research.

In accordance with one embodiment of the present invention the molecular biomarkers determined in the cell culture medium are preferably candidates that are described in the foregoing section.

In another aspect of this invention, in order to assess an anti-angiogenic drug effect, biomarkers can be used separately, in combination with different biomarkers of said invention or with other angiogenesis biomarkers determined outside of this invention.

Additionally, the results of molecular biomarkers evaluation obtained by the determination of the protein level in cell culture media can be accomplished by the gene expression results obtained by the determination of the RNA level of the cell culture cells.

Additionally, the assessment of angiogenesis inhibitor effects by means of molecular biomarkers can be performed by the combined analysis of tissue samples or rather body fluids obtained from an individual treated with integrin inhibitors and the above described cell culture sample treated by integrin inhibitors or rather the cell culture medium thereof.

In a further aspect, the methods as described herein in order to assess the anti-angiogenic effect of integrin inhibitors and the efficacy of said inhibitors on a angiogenesis associated disease can be also applied for the assessment of the anti-angiogenic effect of small molecule ATP site directed multi kinase inhibitors and the efficacy of said inhibitors on a angiogenesis associated disease.

In a preferred embodiment the small molecule ATP site directed multi kinase inhibitor binds and mimicks the ATP-binding site of the target kinase wherein the ATP-binding site is localized in the catalytic active site of the target kinase.

The target kinase of said multi kinase inhibitors preferably comprises different receptor tyrosin kinases such as VEGFRs, PDGFR, KIT, FLT3, CSF-1 and RET as well as the serine/threonine intracellular kinase RAF.

In a notably preferred embodiment, the small molecule ATP site multi kinase inhibitor is sunitinib and/or sorafenib.

Additionally, all methods of said invention for assessing angiogenesis inhibitor effects by means of molecular biomarkers and its use can be beneficial for the prophylaxis and treatment of angiogenesis-associated disease which are described in the background section.

In a further aspect, the invention relates to a method of assessing the effect of a broad range of anti-angiogenic drugs by the use of molecular biomarkers of said invention. Said biomarkers might be used to assess the effect of other anti-angiogenic drugs on angiogenesis which are not included or related to the anti-angiogenic drugs of said invention.

In another aspect, besides the use of said biomarkers for the assessment of angiogenic drugs in the context of a angiogenic therapy they might also be of interest in the early discovery.

The method as specified herein can provide information about the success of an anti-angiogenic therapy in the following way. Said method can be used for the finding and optimizing of an effective dose and/or an effective schedule of an anti-angiogenic drug administered to a patient suffering from an angiogenesis associated disease. In detail, the efficacy of an anti-angiogenic therapy comprising e.g. the treatment of a patient suffering from an angiogenesis-associated disease with an anti-angiogenic drug e.g. an integrin inhibitor in an initial dosis which is assumed to be effective can be assessed by the method of said invention. If the efficacy of said anti-angiogenic therapy seems e.g. to low, altered conditions such as the variation of the dosis and/or the schedule of administration and/or the choice of the anti-angiogenic drug can be applied to said patient. The method of said invention can be repeated for further rounds due to altered anti-angiogenic therapy conditions until the optimum individual therapy for said patient is found.

In another preferred aspect, if the initial dosis of an anti-angiogenic drug administered to a patient suffering from an anti-angiogenic disease is assessed to be effective by the method of said invention, altered conditions might comprise the lowering of a dosis or the stretching of the schedule. A benefit of lowering the dose of the compounds, compositions, agents and therapies administered to an individual includes a decrease in the incidence of adverse effects associated with higher dosages. As a result of the method as claimed and described in more detail in the examples the therapy could be to change the current drug and to replace it by another drug which causes a more distinct biomarker pattern.

Said biomarkers can also be used for the investigation of endothelial cell homeostasis or angiogenesis generally. The evaluation of said biomarkers might give information in experiments which has the scope to get a better understanding of said physiological processes.

In accordance with another aspect of the present invention, the invention provides a method for screening a compound with possible integrin modulating activity. The method comprises
(a) determining the baseline concentration and/or the modification status of at least one selected biomarker in a sample of a body fluid and/or tissue whereby the sample is obtained from an individual that has not been treated with said inhibitor;
(b) determining the concentration and/or the modification status of said selected biomarkers in a sample of (a) that has been treated with said inhibitor.
(c) calculating the differences between the concentrations and/or the modification status of the selected biomarkers obtained by step (a) and (b);
(d) evaluating or predicting from said differences are indicative for a integrin-modulating molecule with effect on angiogenesis and/or antiangiogenic efficacy on angiogenesis associated disease;

In a preferred embodiment, the administration of the compound of interest is performed in different doses which are estimated to be in the range of a possible integrin modulating activity to cells preferably endothelial cells in order to obtain treated cells.

In a preferred embodiment, the cell culture media of said treated cells were taken for the analysis as described in the foregoing sections.

In a preferred embodiment, said biomarkers which are analysed and monitored in cell culture media in response to the treatment with said screening compounds are proteins as described in the foregoing sections.

In a preferred embodiment, the elevation or diminishment of a certain biomarker due to the treatment of screening compounds characterized in certain trials is defined by a p-value not overrunning the value of 0.01.

In another aspect of the present invention said biomarkers preferably proteins are modified in response to the treatment with said screening compounds as described in the foregoing sections.

In another aspect of this invention the change of the gene expression of said biomarkers can be detected on the RNA level by the extraction of the RNA from cells treated by the screening compounds. The expressed RNA transcripts of certain biomarkers can thus be analyzed by known gene expression profiling techniques such as array technology.

Thus, in summary, the invention relates in more detail to the following:
- An ex *vivo* method for assessing the effect of an angiogenesis inhibitor on angiogenesis and/or the efficacy of said inhibitor on an angiogenesis associated disease, by means of at least one selective biomarker, wherein the biomarkers are selected from gene products selected from the group consisting of ADAMTS1, ADAMTS9, SERPINB2, SERPIND2, STC1, ADAM15, SRPX, CORO1C, PBEF1, LTB4DH, IL15RA, ENPP4, PLAUR, LAMA5, TNFRSF1A, CLU, TOR3A, IL1RL1, IL1R1, TCN2, VEGFC, LMNA, CTSL, PTX3, NCL, GUSB, CLEC3B, IL8, CXCL1, SDFR1, STAT1, CDCA7, DAF, HYAL2, CD40, NOTCH1, INHBA, EDN1, MCP1, MMP10, AUTS2, LIPG, PDGFB, BMP4, MGP, HTRA1, FBLN5, HHIP, COL4A1, GALNT, MMP2, IL13RA1, MFAP2, NTN4, DKK3. SERPINE2, COL5A1, CHST1, PGF, EFNA4, BCHE, ENG, ROR1, ANGPTL4, ESM1, CUBN, IGFBP7, CYR61, FLT4, SPOCK, COL12A1, LTBP1, MMP28, TMEM4, CTSB, COL1A2, GRP124, SPARC, FST, DKK4, PHLDB2, PVR, CYR61, MERTK, FSTL3, APLN, PTX3, CTGF, NRG1, DKK3, CD44, BCHE, BDNF, FBXO4, EFEMP1, STC2, IL-6, CRP, CD40 ligand and/or MMP9,
   the method comprising the steps:
   (a) determining the baseline concentration and/or the modification status of at least one selected biomarker in a sample of a body fluid or tissue whereby the sample is obtained from an individual that has not been treated with said inhibitor;
   (b) determining the concentration and/or the modification status of said selected biomarkers in a sample of (a) obtained from an individual that has been treated with said inhibitor or in a sample of (a) that is treated in a subsequent step by said inhibitor.
   (c) calculating the differences between the concentrations and/or the modification status of the selected biomarkers obtained by step (a) and (b);
   (d) assessing or predicting from said differences one or more of the following properties of the inhibitors:
      -- pharmacodynamic effects on the molecular target and on respective signaling pathways linked to the molecular target,
      -- the effect on angiogenesis,
      -- efficacy of treatment and clinical outcome in a patient with an angiogenesis associated disease.
- A corresponding *ex vivo* method, wherein the taken body fluid sample is blood, plasma, serum, lymph, urine, tears, synovial fluid, wound fluid and/or cerebrospinal fluid.
- A corresponding method, wherein the tissue sample comprises a diseased or a healthy tissue.
- A corresponding method, wherein the tissue sample is selected from the group consisting of cancer tissue, skin tissue and/or synovium tissue.
- A corresponding *ex vivo* method, wherein from the taken tissue sample endothelial cells are prepared.
- A corresponding method, wherein the endothelial cells are cultured under laboratory conditions after they have been obtained from an individual and prepared from a tissue sample wherein at least one biomarker is determined from the cell culture medium or extract from said cells.
- A corresponding method, wherein the angiogenesis inhibitor is an integrin inhibitor.
- A corresponding method, wherein the angiogenesis inhibitor is a small molecule ATP site directed multi-kinase inhibitor.
- A corresponding method, wherein the integrin inhibitor is an inhibitor of alpha (V) integrins.
- A corresponding method wherein the inhibitor of alpha (V) integrins is an antibody, or derivative thereof or a small chemical molecule or an RGD peptide.
- A corresponding method wherein the inhibitor of alpha (V) integrins is DI-17E6 or cilengitide.
- A corresponding method wherein the small molecule ATP site directed multi kinase inhibitor is sunitinib and/or sorafenib.
- A corresponding method, wherein the sample of endothelial cells and/or body fluids derive from an healthy and/or diseased individual suffering from a tumor disorder and/or another angiogenesis associated disease.
- A corresponding method, wherein the angiogenesis associated disease is a tumor or a respective disease or disorder thereof.
- A corresponding method wherein the biomarkers are downregulated or upregulated in their level and/or in the modification status due to the administration of the angiogenese inhibitors.
- A corresponding method wherein the biomarkers are selected from the group consisting of ADAMTS1, STC1, MCP-1, IL-8, EDN1, PDGF-BB, INHBA, IL-6, CRP, CD40 ligand and/or MMP9.
- A corresponding method wherein the biomarker ADAMTS1 and/or STC1 is upregulated due to the administration of at least one integrin inhibitors as specified.
- A corresponding method wherein the biomarker MCP1, INHBA, EDN1 and/or PDGF-BB is downregulated due to the administration of one integrin inhibitor as specified.
- A corresponding method wherein the biomarker IL8 is downregulated due to the administration of cilengitide or sorafenib and upregulated due to the administration of DI-17E6 or sunitinib.
- A corresponding method wherein the biomarker IL6 and/or CRP is upregulated due to the administration of DI-17E6.
- A corresponding method wherein the biomarker CD40 ligand is downregulated due to the administration of DI-17E6.
- A corresponding method wherein the biomarker STC1 is downregulated due to the administration of a small molecule ATP directed multi kinase inhibitor as specified.
- A corresponding method wherein the biomarker EDN1 is upregulated due to the administration of a small molecule ATP directed multi kinase inhibitor as specified.
- An ex vivo method for screening integrin modulating compounds in a cell based model, by means of at least one selective biomarker, wherein the biomarkers are selected from gene products selected from the group consisting of
   ADAMTS1, ADAMTS9, SERPINB2, SERPIND2, STC1, ADAM15, SRPX, CORO1C, PBEF1, LTB4DH, IL15RA, ENPP4, PLAUR, LAMA5, TNFRSF1A, CLU, TOR3A, IL1RL1, IL1R1, TCN2, VEGFC, LMNA, CTSL, PTX3, NCL, GUSB, CLEC3B, IL8, CXCL1, SDFR1, STAT1, CDCA7, DAF, HYAL2, CD40, NOTCH1,INHBA, EDN1, MCP1, MMP10, AUTS2, LIPG, PDGFB, BMP4, MGP, HTRA1, FBLN5, HHIP, COL4A1, GALNT, MMP2, IL13RA1, MFAP2, NTN4, DKK3. SERPINE2, COL5A1, CHST1, PGF, EFNA4, BCHE, ENG, ROR1, ANGPTL4, ESM1, CUBN, IGFBP7, CYR61, FLT4, SPOCK, COL12A1, LTBP1, MMP28, TMEM4, CTSB, COL1A2, GRP124, SPARC, FST, DKK4, PHLDB2, PVR, CYR61, MERTK, FSTL3, APLN, PTX3, CTGF, NRG1, DKK3, CD44, BCHE, BDNF, FBXO4, EFEMP1, STC2, IL-6, CRP, CD40 ligand and/or MMP9,
   the method comprising the steps:
   (a) determining the baseline concentration and/or the modification status of at least one selected biomarker in a sample of a body fluid and/or tissue whereby the sample is obtained from an individual that has not been treated with said inhibitor;
   (b) determining the concentration and/or the modification status of said selected biomarkers in a sample of (a) that has been treated with said inhibitor,
   (c) calculating the differences between the concentrations and/or the modification status of the selected biomarkers obtained by step (a) and (b);
   (d) evaluating or predicting from said differences the properties that are indicative for a integrin-modulating molecule with effect on angiogenesis and/or antiangiogenic efficacy on angiogenesis associated disease.
- A diagnostic kit comprising
   containing a detection system for at least one biomarker selected from
   ADAMTS1, ADAMTS9, SERPINB2, SERPIND2, STC1, ADAM15, SRPX, CORO1C, PBEF1, LTB4DH, IL15RA, ENPP4, PLAUR, LAMA5, TNFRSF1A, CLU, TOR3A, IL1RL1, IL1R1, TCN2, VEGFC, LMNA, CTSL, PTX3, NCL, GUSB, CLEC3B, IL8, CXCL1, SDFR1, STAT1, CDCA7, DAF, HYAL2, CD40, NOTCH1,
   INHBA, EDN1, MCP1, MMP10, AUTS2, LIPG, PDGFB, BMP4, MGP, HTRA1, FBLN5, HHIP, COL4A1, GALNT, MMP2, IL13RA1, MFAP2, NTN4, DKK3. SERPINE2, COL5A1, CHST1, PGF, EFNA4, BCHE, ENG, ROR1, ANGPTL4, ESM1, CUBN, IGFBP7, CYR61, FLT4, SPOCK, COL12A1, LTBP1, MMP28, TMEM4, CTSB, COL1A2, GRP124, SPARC, FST, DKK4, PHLDB2, PVR, CYR61, MERTK, FSTL3, APLN, PTX3, CTGF, NRG1, DKK3, CD44, BCHE, BDNF, FBXO4, EFEMP1, STC2, IL-6, CRP, CD40 ligand and/or MMP9
   and an angiogenesis inhibitor as specified which allows to apply the method.
- The use of a biomarker gene or its gene product selected from the preferred group consisting of
   ADAMTS1, STC1, EDN1, MCP1, IL8, PDGF-BB, CRP, CD40 ligand and IL6
   for assessing and predicting the effect of an integrin inhibitor or small molecule ATP site directed kinase inhibitor on angiogenesis in a cell based assay, or the efficacy and / or dosing of such an inhibitor ex vivo.
- The use of a biomarker gene or its gene product selected from the preferred group consisting of
   ADAMTS1, STC1, EDN1, MCP1, IL8, PDGF-BB, CRP, CD40 ligand and IL6
   for the manufacture of an integrin inhibitor or a small molecule ATP site directed kinase inhibitor for the treatment of cancer in patients that are responsive to at least one of these biomarkers.

### DETAILLED DESCRIPTION OF THE INVENTION

The term ***"angiogenesis associated disease"*** contributes to numerous disease or disorders which are directly result from deregulated angiogenesis or biased in an indirect effect by deregulated angiogenesis. These numerous disease include tumor disorders, cardiovascular disease, inflammations, autoimmune disease, degeneration disorders, eye disorders. The deregulated angiogenesis might be induced by diverse pathological stimuli.

The term ***"anti-angiogenic effect"*** means that angiogenesis is blocked or at least inhibited. In the context of this invention, anti-angiogenic drugs or substances are preferably used to antagonize angiogenesis-associated disease by limiting the pathologic formation of new blood vessels (angiogenesis) and the disease symptoms mediated by angiogenesis are ameliorated due to the administration of anti-angiogenic drugs.

The term ***"biomarker"*** refers to a substance that is used as an indicator of a biologic state. It is a characteristic that is objectively measured and evaluated as an indicator of normal biologic processes, pathogenic processes, or pharmacologic responses to a therapeutic intervention. In this invention, the term biomarker is preferably used as a molecular biomarker including gene transcripts such as RNAs and proteins. Said biomarker can be change quantitatively as well as qualitatively. Preferably in this invention, molecular biomarkers change quantitatively, i.e. they are diminished or elevated in their level whereby the p-value characterizing the reliability of a biomarker examined in certain trials under the same conditions does not overrun the value of 0.01.

The term ***"bodyfluids"*** includes fluids that are found in individuals. They include fluids that are excreted or secreted from the body as well as fluids that normally are not. These respective fluids include aqueous humor, blood, serum, interstitial fluid, lymph, mucus, pleural fluid, saliva, plasma, urine, tears, synovial fluid, wound fluid and/or cerebrospinal fluid. Preferably, blood including blood serum and blood plasma and urine are used in this invention.

The terms ***"cancer" and "tumor*/ *tumor disorder"*** refer to or describe the physiological condition in mammals that is typically characterized by unregulated cell growth. By means of the pharmaceutical compositions according of the present invention tumors can be treated such as tumors of the breast, heart, lung, small intestine, colon, spleen, kidney, bladder, head and neck, ovary, prostate, brain, pancreas, skin, bone, bone marrow, blood, thymus, uterus, testicles, cervix, and liver.

The term **"cell based model/ assay"** refers herein to cells which derive from animal or human tissues and/or organs and which are most preferably cultured under laboratory conditions prior to the use for the screening of angiogenesis modulating compounds. A cell based model also includes a cell line.

A ***"derivative"*** of an antibody represents a deduced form of an antibody.

The term ***"diagnostic kit"*** includes preferably a detection system with different packages of diagnostic antibodies and/or gene chip arrays and/or reagents which are necessary for the quantitative and/or qualitative evaluation of the biomarker of this invention and, as a rule, instructions for using the reagents, diagnostic antibodies or gene arrays. The antibodies as well as the reagent can be provided as a liquid, powder, tablet, suspension. The antibodies i.e. gene arrays and reagents may be provided in separate packages suitable for application separately according to the present method. A kit of this invention also contains "instruction for use" of the materials contained in the package.

The term ***"modification status"*** refers to a change of modifications in the gene products such as alterations in the molecular size or glycosilations of proteins. Furthermore, it refers to an change of interactions with different proteins or with same protein subunits.

An ***"individual"*** includes humans and animals which are suffering from an disease/disorder and/or which are healthy as control probands. Furthermore, laboratory animals are included.

A ***"receptor" or "receptor molecule"*** is a soluble or membrane bound / associated protein or glycoprotein comprising one or more domains to which a ligand binds to form a receptor-ligand complex. By binding the ligand, which may be an agonist or an antagonist the receptor is activated or inactivated and may initiate or block pathway signaling.

A ***"tissue sample"*** means a biopsy that is removed from an individual in order to use this material, preferably in terms of cells, for the analysis and evaluation of biomarkers. In this invention, a tissue sample might be used directly after biopsie for the analysis of the biomarkers. In another aspect of this invention, cells extracted from the biopsy material might be cultured under certain laboratory conditions and analyzed for its biomarkers to a later time. In this invention, endothelial cells are preferably used.

The term ***"therapeutically effective dose=TED"*** refers to an amount of a drug e.g. therapeutic antibody effective to treat a disease or disorder in a mammal. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. To the extent the drug may prevent growth and/or kill existing cancer cells, it may be cytostatic and/or cytotoxic. For anti-tumor therapy, efficacy can, for example, be measured by assessing the time to disease progression (TTP) and/or determining the response rate (RR).

The term **"response"** or **"responsiveness"** refers to a condition that can be assessed using any endpoint indication a benefit to a patient, including, without limitation, inhibition to some extent of disease progression, including slowing down a complete arrest; reduction in the number of disease episodes and/or symptoms; decrease of auto-immune responses against the applied inhibitor drug; increase in the length of disease-free presentation following treatment; and decreased mortality at a given point of time following treatment. The term refers to a measurable response including complete response (CR) and partial response (PR).

**Murine, chimeric** or **humanized versions** of an antibody in the context of this invention include equivalent antibodies or derivatives wherein portions of antibody are substituted according to the definitions of murine antibodies, chimeric antibodies or humanized antibodies.

In this study, biomarkers linked to the anti-angiogenic activity of integrin inhibitors and small molecule ATP site directed multi kinase inhibitors that might be accessible in body fluids and therefore might allow analysis of target modulation in non-invasive way were identified. For this, two different strategies were pursued. Firstly, genome-wide changes in gene expression profiles of endothelial cells in response to drug treatment were analyzed. Secondly, a candidate-proteomic driven approach using an *in vivo* angiogenesis model to identify drug-regulated plasma biomarkers was performed.

### 1. Identification of biomarkers using gene expression profiles

To identify biomarkers associated with treatment, HUVEC were treated with cilengitide or DI-17E6. Western blot analysis revealed that following treatment phosphorylation of FAK and MAPK was inhibited for cilengitide, and for DI-17E6, respectively. Gene expression profiles were generated from experiments with each condition being tested in triplicates. For this, RNA was extracted from treated and control cells and hybridized to genome-wide Affymetrix oligonucleotide microarrays to generate gene expression profiles. After preprocessing with the GCRMA algorithm (Wu Z, Journal American Statistical Association 2004; 99: 909-917), Affymetrix GeneChip data were filtered to exclude probe sets with low variability, low signal or poor probe annotation. Genes were specifically filtered to obtain secreted candidates that might be detectable in body fluids in humans and could be monitored in a non-invasive way in patients. Differentially expressed genes between drug-treated cells and non-treated or control-treated cells were identified by linear model analysis of the expression data of each probe set, followed by moderated t-tests (Smyth GK, Statistical Applications in Genetics and Molecular Biology 2004; 3: Article 3).
- Genes up-regulated in response to DI-17E6 include ADAMTS1, ADAMTS9, SERPINB2, SERPIND2, STC1, ADAM15, SRPX, CORO1C, PBEF1, LTB4DH, IL15RA, ENPP4, PLAUR, LAMA5, TNFRSF1A, CLU, TOR3A, IL1RL1, IL1R1, TCN2, VEGFC, LMNA, CTSL, PTX3, NCL, GUSB, CLEC3B, IL8, CXCL1, SDFR1, STAT1, CDCA7.
- **Genes up-regulated in response to Cilengitide include** ADAMTS9, DAF, STC1, ADAMTS1, LAMA5, TCN2, SRPX, ADAM15, GUSB, HYAL2, PLAUR, CD40, NOTCH1, ENPP4.
- **Genes down-regulated in response to DI-17E6 include** INHBA, EDN1, MCP1, MMP10, AUTS2, LIPG, PDGFB, BMP4, MGP, HTRA1, FBLN5, HHIP, COL4A1, GALNT, MMP2, IL13RA1, MFAP2, NTN4, DKK3. SERPINE2, COL5A1, CHST1, PGF, EFNA4, BCHE, ENG, ROR1, ANGPTL4, ESM1, CUBN, IGFBP7, CYR61, FLT4, SPOCK, COL12A1, LTBP1, MMP28, TMEM4, CTSB, COL1A2, GRP124, SPARC.
- **Genes down-regulated in response to cilengitide include** INHBA, EDN1, FST, DKK4, BMP4, MCP1, HHIP, NTN4, PHLDB2, PVR, CYR61, AUTS2, MERTK, FSTL3, APLN, PTX3, ANGPTL4, CTGF, SPOCK, IL8, NRG1, DKK3, CD44, BCHE, BDNF, FBXO4, EFEMP1, STC2.

**Genes which show under the given condition an extraordinary response / response change / responsiveness are underlined.**

The genes and gene products of said genes comprise by definition according to this invention also homologues sequences eliciting the same functional activity; especially sequences are concerned which have a homology to said original sequences of more than 80%, preferably more than 90% and especially more than 95%. Thus, sequences having the following homologies are subject-matter of the invention: 80%, 81%, 82%, 83%, 84%, 85%, 86%, 87%, 88%, 89%, 90%, 91%, 92%, 93%, 94%, 95%, 96%, 97%, 98%, and 99%.

Genes that were highly significantly regulated on the RNA level by either integrin inhibitor were analysed on the protein level. Since secreted biomarkers are relevant, the individual candidates were analysed in supernatants of integrin-inhibitor treated cells. HUVEC were treated either DI-17E6 or cilengitide. After different times, culture supernatants were collected and analysed for different biomarker candidates by Western blotting, ELISA or Luminex. Drug-induced biomarker regulation was evaluated in comparison to control cells. Among the different candidates, ADAMTS1, STC-1, MCP-1, IL-8, Endothelin-1, activin A and PDGF-BB were found to be regulated in response to integrin inhibitors.

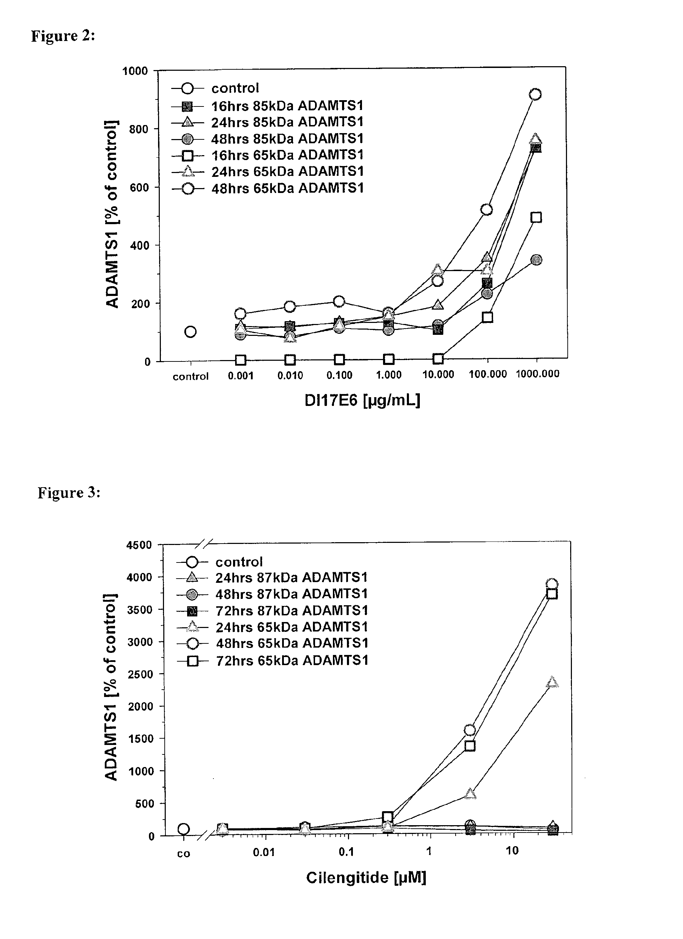

Western blot analyses revealed that ADAM metallopeptidase with thrombospondin type 1 motif **(ADAMTS1)** and Stanniocalcin-1 **(STC-1)** were up-regulated in response to treatment with DI-17E6 or cilengitide (Figure 2-4). ADAMTS1 is a metalloprotease residing in the extracellular space. Using different biological systems, it has been demonstrated that ADAMTS1 inhibits angiogenesis (Vazquez F. et al., J Biol Chem 1999; 274(33): 23349-57). STC-1 is a secreted glycoprotein that has originally been described as hormone involved in calcium and phosphate homeostasis in bony fishes. It plays a role in many physiological processes including angiogenesis and both pro- and anti-angiogenic activities have been described for STC-1 (Filvaroff EH. Et al., Endocrinology 2002; 143: 3681-90; Zlot C. et al., J Biol Chem. 2003; 278(48):47654-9).

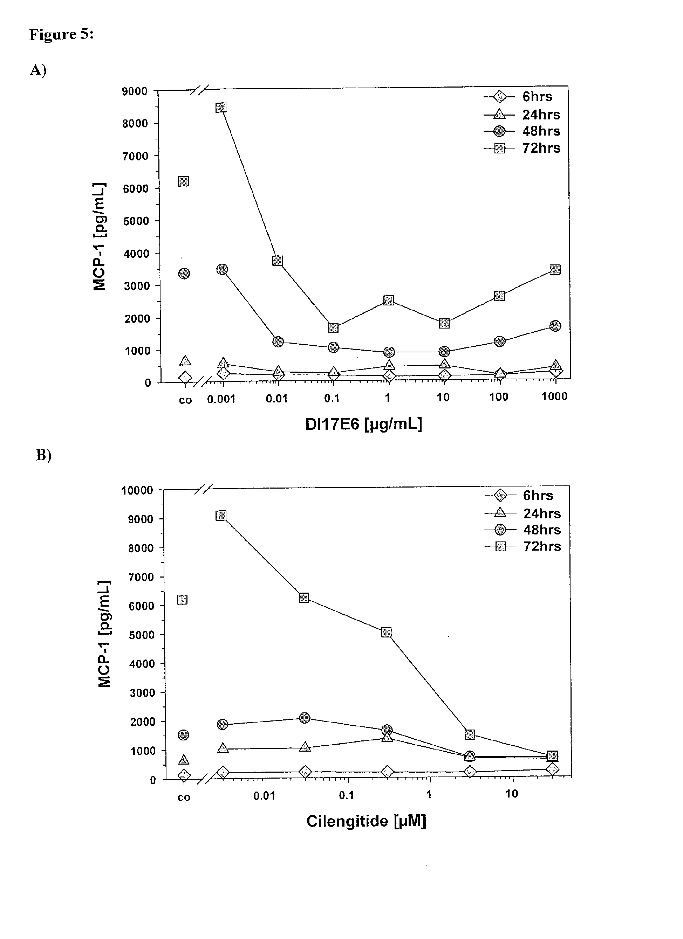

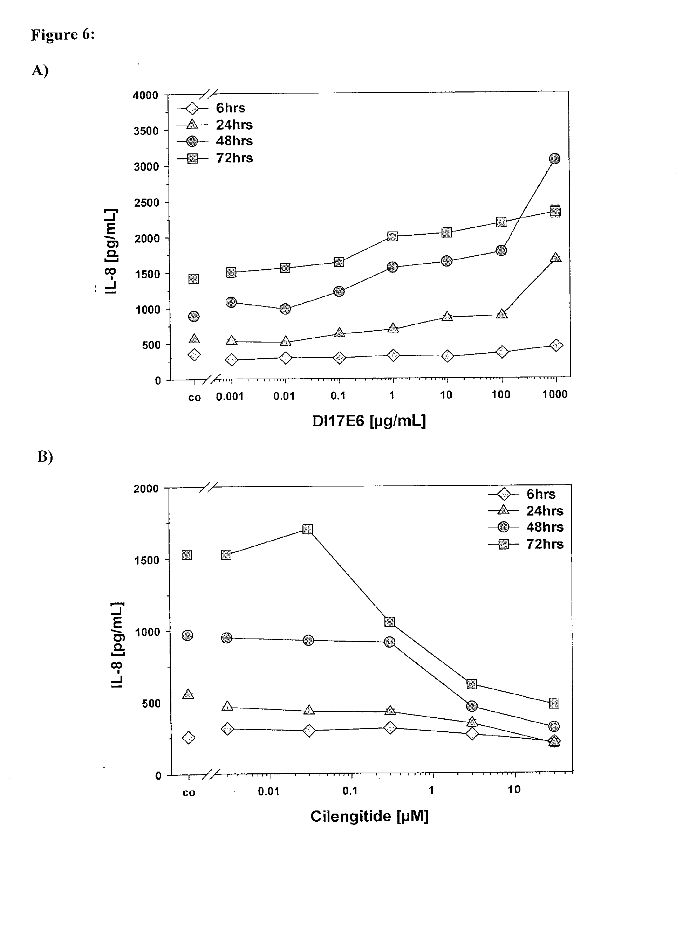

Monocyte chemoattractant protein-1 **(MCP-1/CCL2)** and Interleukin-8 **(IL-B/CXCLB)** were analysed by Luminex. MCP-1 was down-regulated in response to DI-17E6 and cilengitide (Figure 5). In contrast, **IL-8** was differentially regulated by both integrin inhibitors. In response to DI-17E6 IL-8 was up-regulated, whereas cilengitide induced a down-regulation of this chemokine (Figure 6). MCP-1 plays a critical role in the recruitment and activation of macrophages. It is one of the key agonists to attract pro-angiogenic macrophages to tumors. Inhibition of MCP-1 reduces angiogenesis and tumor growth in mice (Koga M. et al., Biochem Biophys Res Commun. 2008; 365(2): 279-84). IL-8/CXCL8 was initially characterized for its leukocyte chemotactic activity, it is known to possess tumorigenic and pro-angiogenic properties (Xie K et al., Cytokine Growth Factor Rev 2001; 12(4): 375-91).

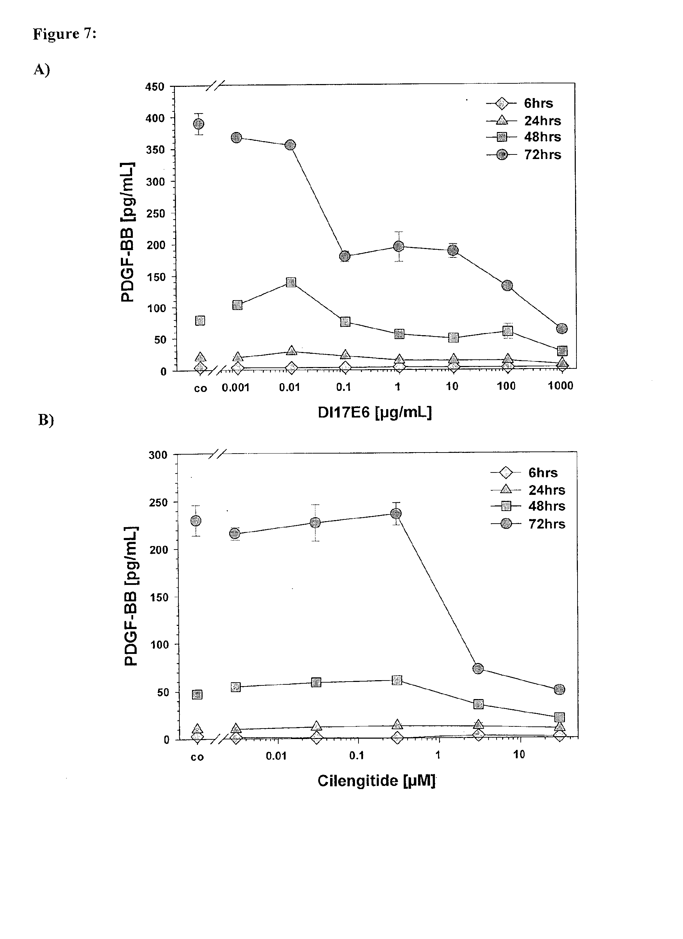

**Activin A,** endothelin-1 **(EDN1)** and platelet-derived growth factor BB **(PDGF-BB)** were analysed by ELISA. All three proteins were down-regulated in response to DI-17E6 and cilengitide (Figure 7-9). EDN1 mediates multiple tumor-promoting activities including angiogenesis. It regulates Integrin-linked kinase (ILK), a multidomain focal adhesion protein conveying intracellular signaling elicited by integrins (Rosanò L. et al., Mol Cancer Ther. 2006; 5(4):833-42). Activin A, a homodimer of two inhibin ßA subunits, is a member of the activin/inhibin family, which belongs to the transforming growth factor-ß (TGF-ß) superfamily. It exerts a large spectrum of biological activities including developmental and reproductive processes, inflammation, cancer and angiogenesis. The role of activin A in the regulation of angiogenesis is contrive, since both pro- and anti-angiogenic functions have been described (Panopoulou E et al., Cancer Res. 2005; 65(5):1877-86; Poulaki V. et al., Am J Pathol. 2004; 164(4): 1293-302). PDGF-BB is one of four PDGF ligands, PDGF-A, PDGF-B, PDGF-C and PDGF-D (Fredriksson L et al., Cytokine Growth Factor Rev. 2004; 15: 197-204). It belongs to the VEGF/PDGF superfamily of ligands and receptors, grouped together on the basis of their close sequence homology and evolutionary relationship. PDGF-BB is frequently expressed at high levels in various tumor tissues (Nguyen M. et al., J. Natl. Cancer Inst.1994; 86:356-361). It is a mitogenic and chemotactic factor for pericytes and plays crucial role in regulating vascular cells during blood vessel wall assembly (Lindahl P et al., Science 1997; 277: 242-245).

The regulation of the identified biomarkers in response to other different angiogenesis inhibitors, Sunitinib and Sorafenib, to find out if the identified biomarkers correlate generally with angiogenesis inhibition was analyzed. Both inhibitors are small molecule multi-kinase inhibitors that operate via unrelated mechanism of action by binding to the ATP-binding catalytic site of kinases (Morabito A et al., Current status and Future oncologist 2006; 11: 753 - 764).

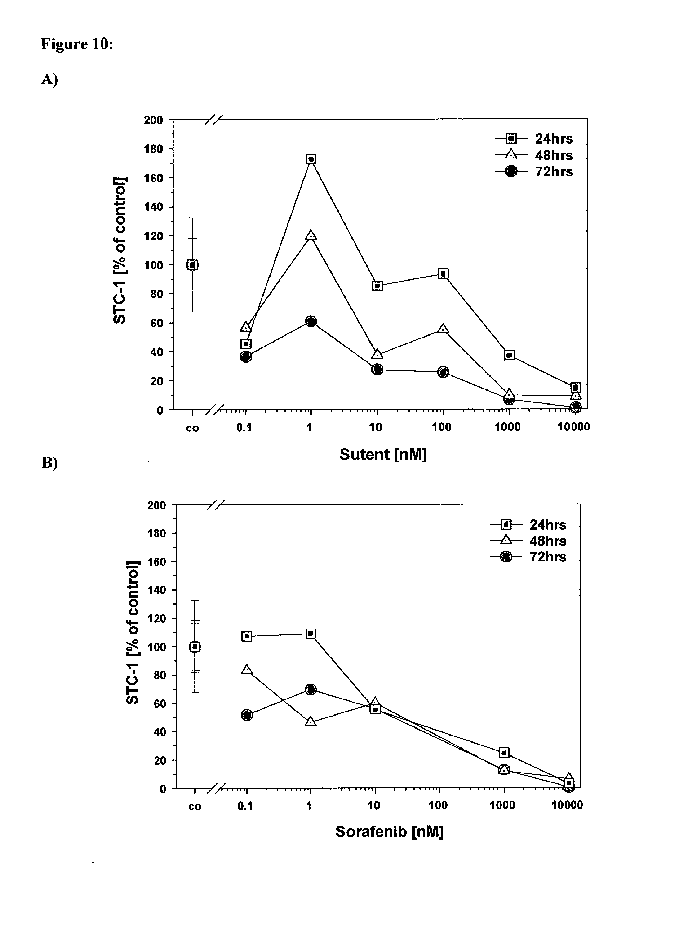

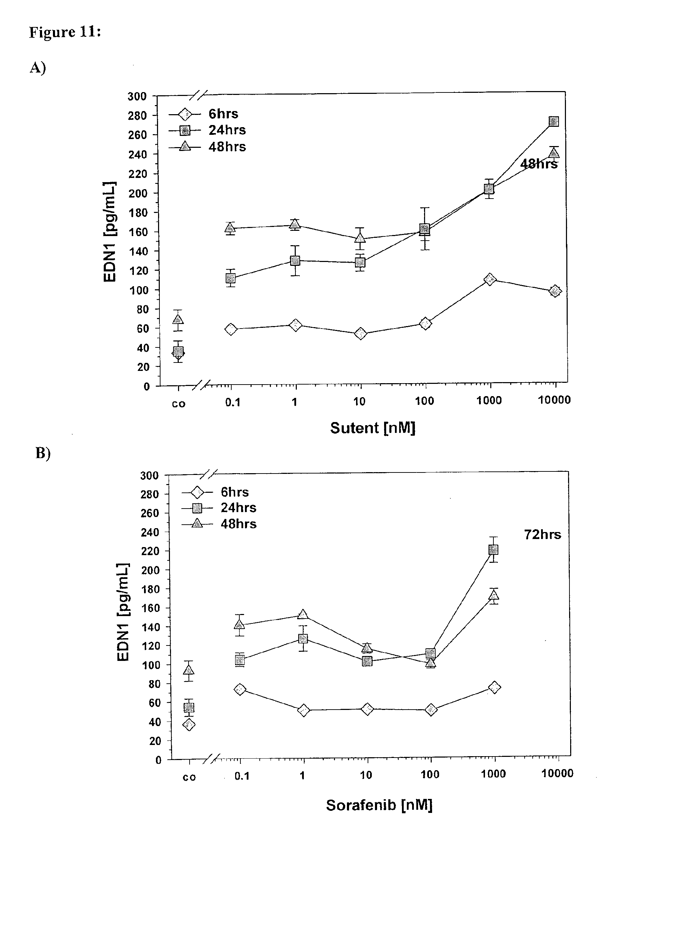

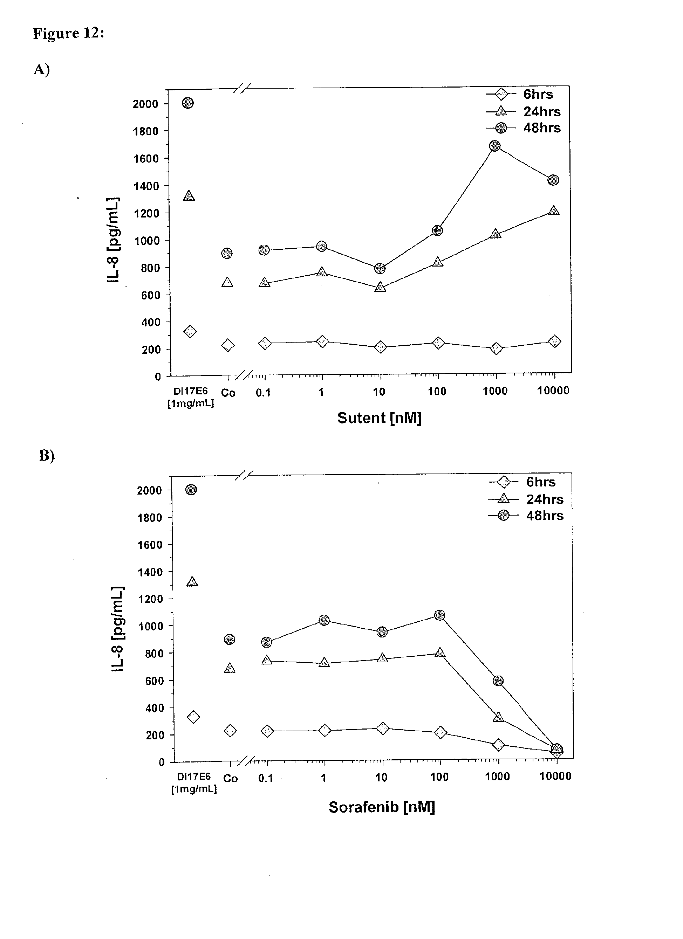

Levels of biomarker candidates were analyzed in endothelial cell supernatants following treatment with Sunitinib (Sutent) and Sorafenib. As observed for integrin inhibitors several biomarkers were regulated in response to these kinase inhibitors. For example, STC-1 was down-regulated in response to either drug (Figure 10), whereas EDN1 was up-regulated (Figure 11). Interestingly, an inverse regulation was observed for integrin inhibitors. IL-8 was differentially regulated by either kinase inhibitors. Sutent caused an up-regulation of this chemokine, whereas Sorafenib reduced protein levels following treatment (Figure 12). In case of integrin inhibitors a differential regulation was also observed. Together these data show that the identified biomarkers might be used to monitor activity of integrin inhibitors as well as kinase inhibitors. However, distinct effects on endothelial cell homeostasis might be expected since the respective proteins are regulated in different ways and might result in distinct biological responses. Since all drugs operate via a distinct mechanism of action the pathways implicated in regulating the different biomarkers likely differ between the different inhibitors.

In summary, drug-specific biomarkers correlating with pharmacodynamic effects of integrin inhibitors have been identified in gene expression profiles in endothelial cultures. Several of the identified candidates were confirmed as drug-regulated proteins in cellular supernatants indicating that they might be detectable in body fluids. Some of the candidates were also regulated by unrelated multi-kinase inhibitors suggesting that the biomarkers might be applied in a broad sense to monitor effects on endothelial cell homeostasis and angiogenesis.

### 2. Identification of drug-regulated plasma biomarkers by means of an in vivo angiogenesis model

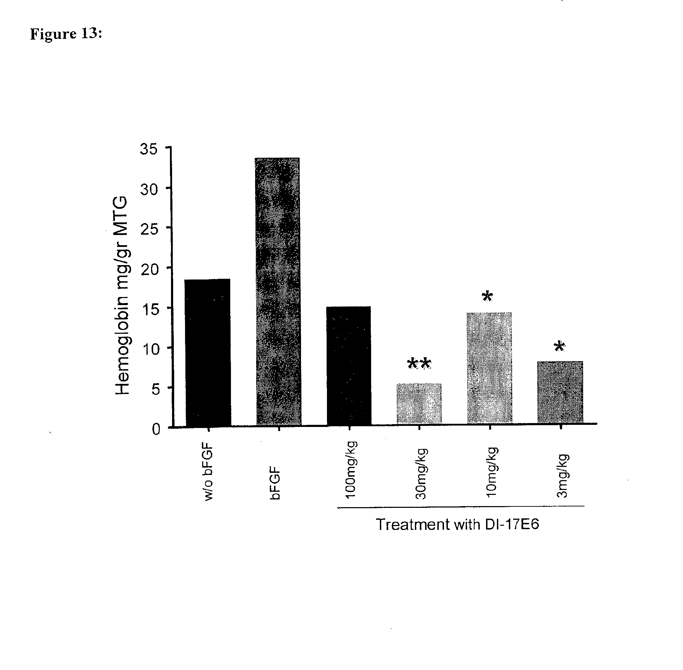

In this model, angiogenesis was induced in matrigel plugs by the angiogenic growth factor bFGF. Matrigel plugs containing bFGF were implanted subcutan into the abdomen of healthy Cynomologus monkeys, and the animals were injected with DI-17E6 i.v. at different doses immediately thereafter. Evaluation of angiogenesis was carried out after matrigel implantation by quantification of the hemoglobin content in the matrigel plugs. Each condition was tested in different animals implanted with matrigel plugs. It was found that treatment of monkeys with DI-17E6 blocked the formation of new blood vessel at all doses (Figure 13).

To identify biomarkers correlating with PD effects of DI-17E6, plasma samples were collected before treatment and after treatment. Absolute concentrations of different proteins were measured by Luminex by comparing to respective standards. Raw data were preprocessed by log-transformation and the effect of DI-17E6 was examined in a t-test analysis by comparing DI-17E6-treated groups to placebo-treated animals at each timepoint.

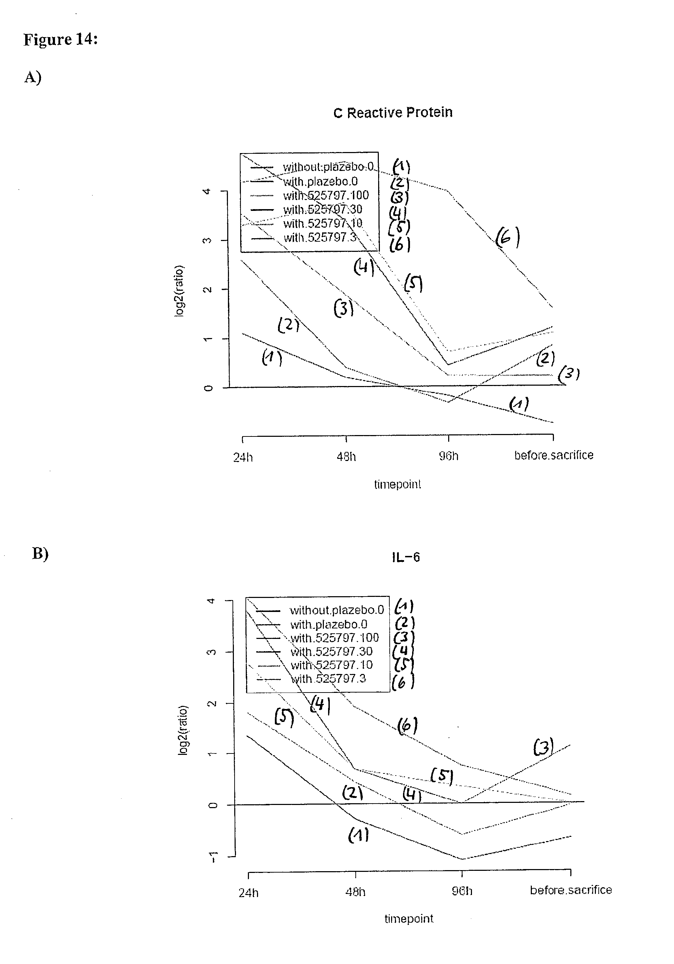

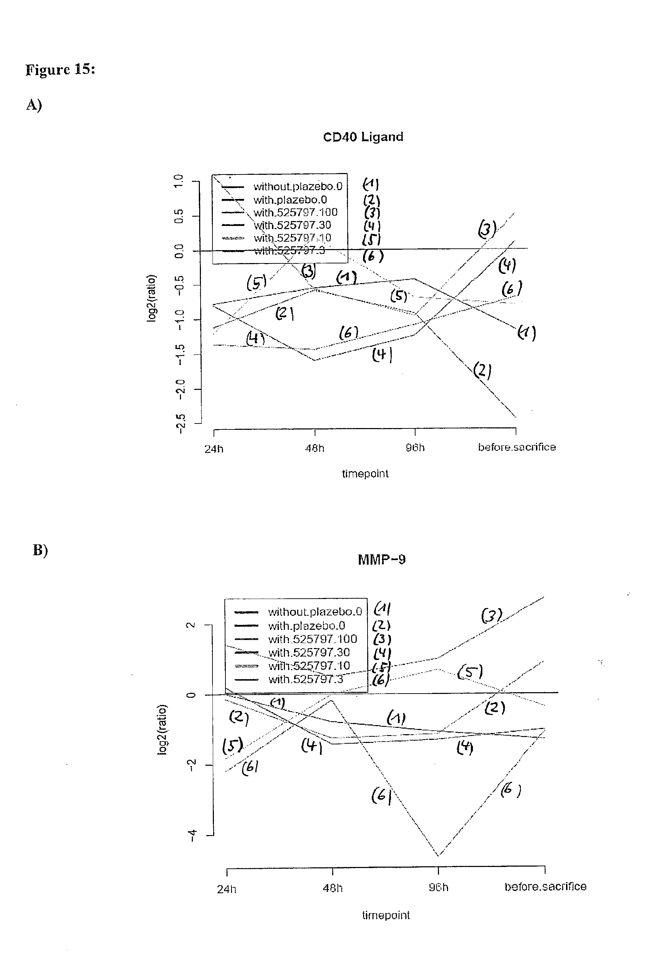

Proteins that were significantly regulated include C-reactive protein (CRP) and IL-6, CD40 ligand and MMP9. CRP and IL-6 were up-regulated in response to treatment with DI-17E6 (Figure 14). In contrary, CD40 ligand and MMP9 were specifically regulated in the control group. After 144h, CD40 ligand was down-regulated and MMP9 was up-regulated in placebo treated animals (Figure 15). CRP is a marker of inflammation and a key mediator of the acute-phase response (Pepys MB et al., Adv Immunol 1983; 34: 141-212). It recognizes damaged cells, initiates their elimination by interacting with the immune system and might thus be implicated in removing cells that have been damaged in response to DI-17E6 treatment. IL-6 is a pleiotropic cytokine associated with angiogenesis and tumor growth (Keller ET et al., Front. Biosci. 1996. 1: 340-357 and Ancrile B et al., Genes Dev. 2007 Jul; 21 (14): 1714-9). Up-regulation of IL-6 in response to DI-17E6 treatment seems conflicting with its pharmacological activity, but might reflect a regulatory feedback loop as it has been observed for other angiogenesis inhibitors. CD40 ligand is a transmembrane protein structurally related to tumor necrosis factor-α. It is a pro-inflammatory and pro-coagulant factor contributing to inflammation, thrombosis and neoangiogenesis (Ferroni P et al., Curr Med Chem. 2007; 14(20):2170-80). Lastly, MMP9 belongs to the family of matrix metalloproteinases that plays a role in mediating homeostasis of the extracellular environment. Both pro- and anti-tumorigenic activities have been described (Egeblad M et al., Nature Rev. Cancer 2002; 2:161-174). It modulates neovessel remodeling and promotes tumor growth (Seandel M et al., Cancer Cell 2008; 13(3):181-3).

In summary, drug-specific pharmacodynamic plasma biomarkers have been detected that could be detectable in body fluids in humans and might be used to provide proof of concept for target modulation; select appropriate dose and schedule; and potentially explain or predict clinical outcomes. Of the identified biomarkers, CRP and MMP9 might be mechanistically involved in blocking angiogenesis following DI-17E6 treatment.

### 3. Summary

In these two studies several PD biomarkers for integrin inhibitors and multi kinase inhibitors have been identified that might be detectable in body fluids in humans and could be used to provide proof of concept for target modulation; provide rational selection of dose and schedule; and potentially explain or predict clinical outcomes. Interestingly, most candidates have been associated with angiogenesis and might thus be mechanistically involved in blocking angiogenesis following drug treatment. Interestingly, some biomarkers were regulated by unrelated drugs with different mechanism of action suggesting that they could be used to monitor effects on endothelial cell homeostasis and angiogenesis generally, and thus are suitable to be applied for different drug classes, including integrin inhibitors (such as cilengitide, DI-17E6), VEGF inhibitors (such as Avastin), multi-kinase inhibitors (such as Sunitinib, Sorafenib).

### SHORT DESCRIPTION OF THE FIGURES:

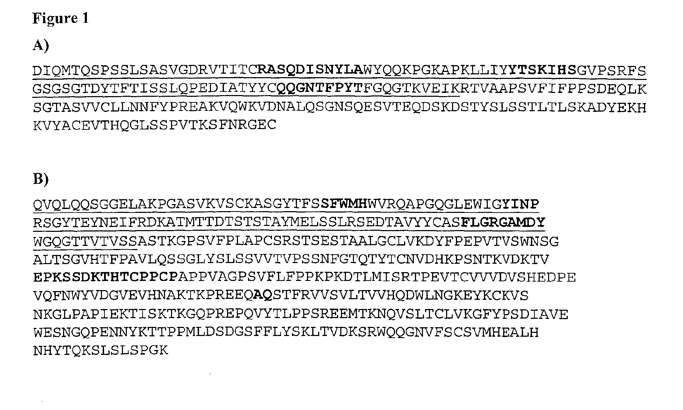

**Figure 1****:** shows complete light (A) and heavy (B) chain sequences of DI-17E6 Variable regions are underlined with CDR's in bold. Bold sequences in constant region indicate modified IgG1 hinge and mutation at positions 296 and 297.
**Figure 2**: presents that DI-17E6 induces levels of ADAMTS1 protein in HUVEC supernatants
**Figure 3****:** depicts that cilengitide induces levels of ADAMTS1 protein in HUVEC supernatants
**Figure 4****:** depicts that DI-17E6 and cilengitide induce protein levels of STC-1 in HUVEC supernatants
**Figure 5****:** presents regulation of MCP-1 in HUVECs in response to DI-17E6 and cilengitide
**Figure 6****:** depicts regulation of IL-8 in HUVECs in response to DI-17E6 and cilengitid.
**Figure 7****:** depicts regulation of PDGF-BB in HUVECs in response to DI-17E6 and cilengitid.
**Figure 8****:** presents regulation of EDN1 in HUVECs in response to DI-17E6 and cilengitide.
**Figure 9****:** presents regulation of activin A in HUVECs in response to DI-17E6 and cilengitid.
**Figure 10****:** presents regulation of STC-1 in HUVECs in response to sutent and sorafenib
**Figure 11****:** presents regulation of EDN1 in HUVECs in response to sutent and sorafenib
**Figure 12****:** presents regulation of IL-8 in HUVECs in response to sutent and sorafenib
**Figure 13****:** presents DI-17E6 inhibition of angiogenesis in matrigel plugs in monkeys
**Figure 14****:** presents regulation of plasma CRP and IL-6 in response to treatment with DI-17E6
**Figure 15****:** presents regulation of plasma CD40 Ligand and MMP9 in response to treatment with DI-17E6
**Figure 16****:** presents inhibition of integrin-specific signalling pathways

### EXAMPLES

### Example 1:

### DI-17E6 induces levels of ADAMTS1 protein in HUVEC supernatants

HUVECs were cultured o/n in vitronectin-coated dishes and incubated with different concentration of DI-17E6 for 6h to 72h. Culture supernatants were collected analysed for ADAMTS1 by Western blotting. A) DI-17E6 mediated regulation of the of ADAMTS1 (65kD and 85kD) (Figure 2).

### Example 2:

### Cilengitide induces levels of ADAMTS1 protein in HUVEC supernatants

HUVECs were cultured o/n in vitronectin-coated dishes and incubated with different concentration of cilengitide for 6h to 72h. Culture supernatants were collected analysed for ADAMTS1 by Western blotting. A) Cilengitide mediated regulation of ADAMTS1 (65kD and 85kD). (Figure 3).

### Example 3:

### DI-17E6 and cilengitide induce protein levels of STC-1 in HUVEC supernatants

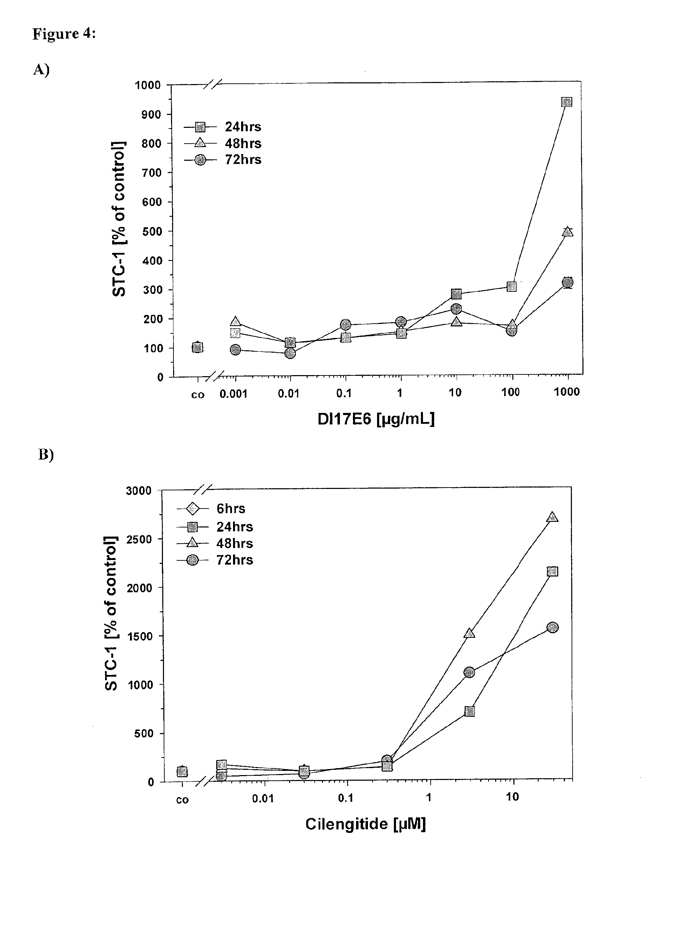

HUVECs were cultured o/n in vitronectin-coated dishes and incubated with different concentration of DI-17E6 or cilengitide for 24h to 72h. Culture supernatants were collected analysed for STC-1 by Western blotting. A) Regulation of STC-1 in response to DI-17E6. B) Regulation of STC-1 in response to cilengitide (Figure 4).

### Example 4:

### Regulation of MCF-1 in HUVECs in response to DI-17E6 and cilengitide

HUVECs were cultured o/n in vitronectin-coated dishes and incubated with different concentration of DI-17E6 or cilengitide for 6h to 72h. Culture supernatants were collected analysed for MCP-1 by Luminex. A) Regulation of MCP-1 in response to DI-17E6. B) Regulation of MCP-1 in response to cilengitide (Figure 5).

### Example 5:

### Regulation of IL-8 in HUVECs in response to DI-17E6 and cilengitide

HUVECs were cultured o/n in vitronectin-coated dishes and incubated with different concentration of DI-17E6 or cilengitide for 6h to 72h. Culture supernatants were collected analysed for IL-8 by Luminex. A) Regulation of IL-8 in response to DI-17E6. B) Regulation of IL-8 in response to cilengitide (Figure 6).

### Example 6:

### Regulation of PDGF-BB in HUVECs in response to DI-17E6 and cilengitide

HUVECs were cultured o/n in vitronectin-coated dishes and incubated with different concentration of DI-17E6 or cilengitide for 6h to 72h. Culture supernatants were collected analysed for PDGF-BB by ELISA. A) Regulation of PDGF-BB in response to DI-17E6. B) Regulation of PDFG-BB in response to cilengitide (Figure 7).

### Example 7:

### Regulation of EDN1 in HUVECs in response to DI-17E6 and cilengitide

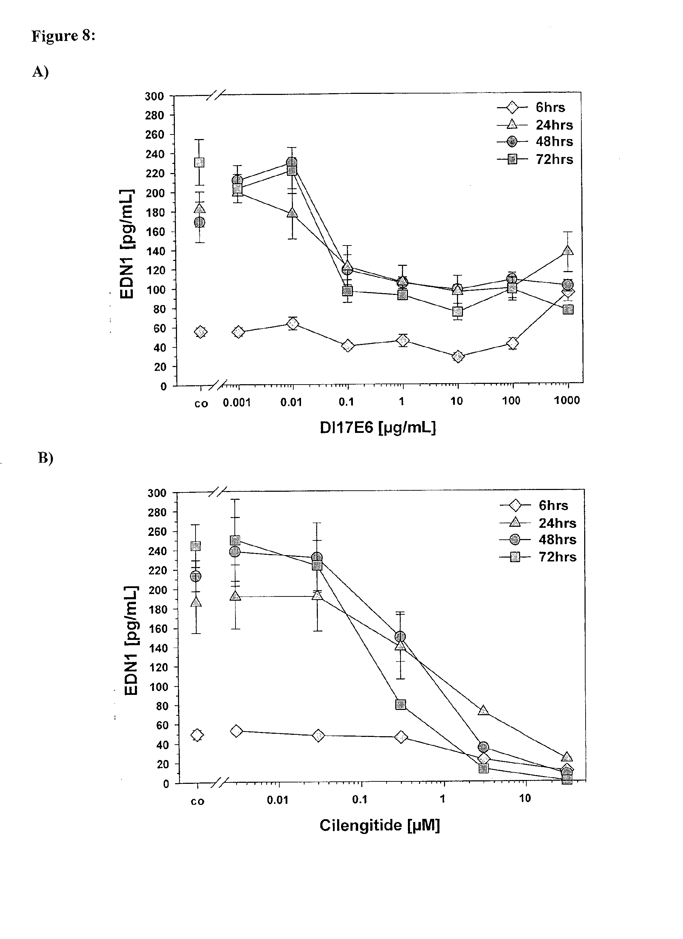

HUVECs were cultured o/n in vitronectin-coated dishes and incubated with different concentration of DI-17E6 or cilengitide for 6h to 72h. Culture supernatants were collected analysed for EDN1 by ELISA. A) Regulation of EDN1 in response to DI-17E6. B) Regulation of EDN1 in response to cilengitide (Figure 8).

### Example 8:

### Regulation of activin A in HUVECs in response to DI-17E6 and cilengitide

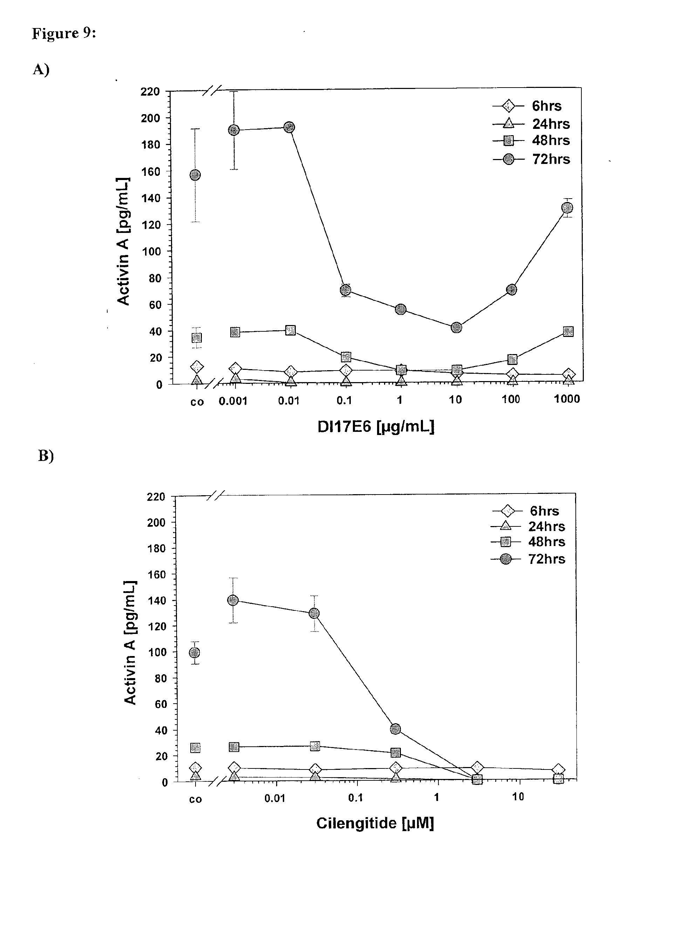

HUVECs were cultured o/n in vitronectin-coated dishes and incubated with different concentration of DI-17E6 or cilengitide for 6h to 72h. Culture supernatants were collected analysed for activin A by ELISA. A) Regulation of activin A in response to DI-17E6. B) Regulation of activin A in response to cilengitide (Figure 9).

### Example 9:

### Regulation of STC-1 in HUVECs in response to Sutent and Sorafenib

HUVECs were cultured o/n in vitronectin-coated dishes and incubated with different concentration of Sutent or Sorafenib for 6h to 48h. Culture supernatants were collected analysed for STC-1 by Western blotting. A) Regulation of STC-1 in response to Sutent. B) Regulation of STC-1 in response to Sorafenib (Figure 10).

### Example 10:

### Regulation of EDN1 in HUVECs in response to Sutent and Sorafenib

HUVECs were cultured o/n in vitronectin-coated dishes and incubated with different concentration of Sutent or Sorafenib for 6h to 48h. Culture supernatants were collected analysed for EDN1 by ELISA. A) Regulation of EDN1 in response to Sutent. B) Regulation of EDN1 in response to Sorafenib (Figure 11).

### Example 11:

### Regulation of IL-8 in HUVECs in response to Sutent and Sorafenib

HUVECs were cultured o/n in vitronectin-coated dishes and incubated with different concentration of Sutent or Sorafenib for 6h to 48h. Culture supernatants were collected analysed for IL-8 by Luminex. A) Regulation of IL-8 in response to Sutent. B) Regulation of IL-8 in response to Sorafenib (Figure 12).

### Example 12:

### DI-17E6 inhibits angiogenesis in matrigel plugs in monkeys

Inhibition of angiogenesis in Matrigel plugs in monkeys by DI-17E6. Angiogenesis was induced in subcutaneous matrigel implants containing 5 µg/mL of bFGF in Cynomolgus monkeys. DI-17E6 was given as a single infusion at 100, 30, 10, or 3 mg/kg. After 6 days, angiogenesis was quantified by measuring the hemoglobin content in the matrigel plugs. * denotes a p-value < 0.05 and ** denotes a p-value <0.01 (Figure 13).

### Example 13:

### Regulation of plasma CRP and IL-6 in response to treatment with DI-17E6

Cynomolgus monkeys were implanted with bFGF-containing matrigel plugs and with different concentration of DI-17E6 for 6 days. Plasma was collected and analysed for CRP and IL-6 by Luminex. A) Regulation of CRP. B) Regulation of IL-6 (Figure 14).

### Example 14:

### Regulation of plasma CD40 Ligand and MMP9 in response to treatment with DI-17E6

A) Cynomolgus monkeys were implanted with bFGF-containing matrigel plugs and with different concentration of DI-17E6 for 6 days. Plasma was collected and analysed for CD40 Ligand and MMP9 by Luminex. A) Regulation of CD40 Ligand. B) Regulation of MMP9 (Figure 15).

### Example 15:

### Inhibition of integrin-specific signalling pathways:

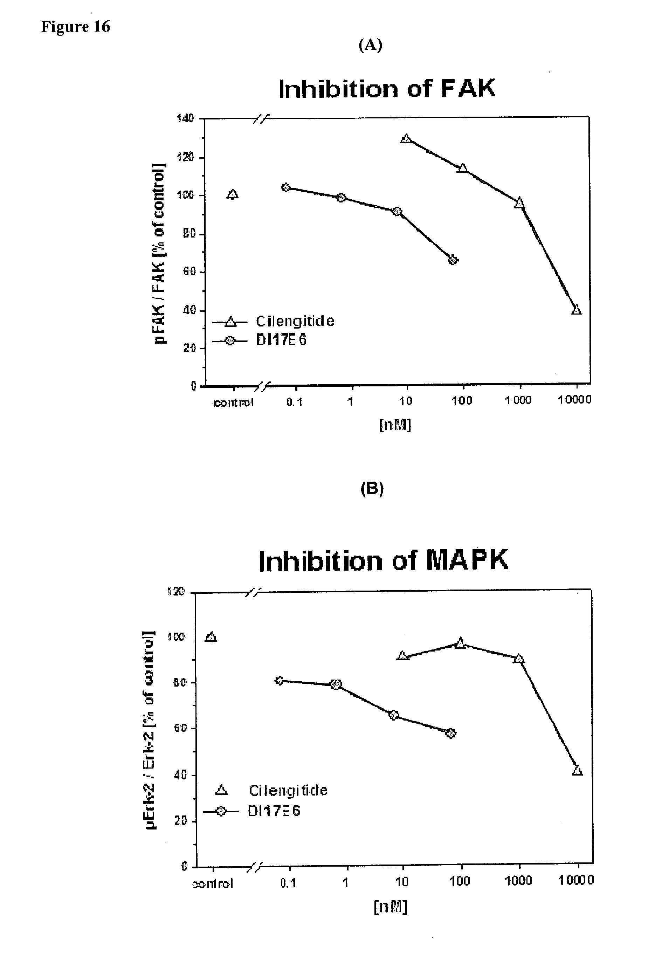

Besides growth factor stimulation, integrin-mediated adhesion to the extracellular matrix is important to promote cell proliferation, survival, and migration. Upon integrin ligation a wide range of intracellular signaling events are induced, including activation of Ras, MAPK, FAK, Src, Rac/Rho/cdc42 GTPases. To support survival of endothelial cells during angiogenesis, integrins alpha(V) beta(3) / (5) together with FAK are required for a sustained activation of the MAPK cascade (i.e. ERK1/2). In this study the effect of two integrin inhibitors, DI-17E6 and cilengitide, on integrin-mediated signaling pathways in endothelial cells has been analyzed. For this purpose, human umbilical vein endothelial cells (HUVEC) were plated on vitronectin to activate alpha(V)beta(3) integrin, and then treated with culture medium or different concentrations of DI-17E6 or cilengitide. After 24h of treatment, cell lysates were prepared and analysed by Western blotting. Effects on signaling pathways were evaluated by comparing the ratio of phosphorylated to total levels of the respective signaling molecule in order to account for potential effects arising from cell detachment. As seen, DI-17E6 at concentrations ranging from 0.01µg/mL to 10µg/mL and cilengitide at concentrations ranging from 0.01µM to 10µM blocked activation of FAK and ERK1/2 in a dose-dependent way (Figure 16).

## Claims

1. The integrin inhibitor DI17E6 (abituzumab) for use in a method of treatment and / or diagnosis of a patient suffering from a tumor disorder and / or another angiogenesis associated disease, wherein the treatment is therapeutically effective and responsive to the patient, when in a sample of a body fluid or a tissue of the patient the expression of at least one selective biomarker gene or gene product thereof selected from the group consisting of ADAMST1, STC1, IL8, IL6, EDN1, MCP1, PDGF-BB, CRP, CD40 ligand is up- or down regulated, and the biomarker genes or gene products ADAMTS1, STC1, IL8, IL6 and CRP are upregulated and the biomarker genes or gene products MCP1, EDN1, PDGF-BB and CD40 ligand are downregulated after administration of the inhibitor.

2. The integrin inhibitor and RGD peptide cilengitide for use in a method of treatment and / or diagnosis of a patient suffering from a tumor disorder and / or another angiogenesis associated disease, wherein the treatment is therapeutically effective and responsive to the patient, when in a sample of a body fluid or a tissue of the patient the expression at least one selective biomarker gene or gene product thereof selected from the group consisting of ADAMST1, STC1, IL8, IL6, EDN1, MCP1, PDGF-BB, CRP, CD40 ligand is up- or down regulated,of the biomarker genes or gene products ADAMTS1 and STC1 are upregulated, and the biomarker genes or gene products MCP1, EDN1, PDGF-BB, and IL-8 are downregulated after administration of the inhibitor.

3. The receptor tyrosine kinase inhibitor sutinib (sutent) for use in a method of treatment and / or diagnosis of a patient suffering from a tumor disorder and / or another angiogenesis associated disease, wherein the treatment is therapeutically effective and responsive to the patient, when in a sample of a body fluid or a tissue of the patient the expression of at least one selective biomarker gene or gene product thereof selected from the group consisting of ADAMST1, STC1, IL8, IL6, EDN1, MCP1, PDGF-BB, CRP, CD40 ligand is up- or down regulated, and the biomarker genes or gene products EDN1 and II-81 are upregulated, and the biomarker gene or gene product of STC-1 is downregulated after administration of the inhibitor.

4. The multi-kinase inhibitor sorafenib for use in a method of treatment and / or diagnosis of a patient suffering from a tumor disorder and / or another angiogenesis associated disease, wherein the treatment is therapeutically effective and responsive to the patient, when in a sample of a body fluid or a tissue of the patient the expression of at least one selective biomarker gene or gene product thereof selected from the group consisting of ADAMST1, STC1, IL8, IL6, EDN1, MCP1, PDGF-BB, CRP, CD40 ligand is up- or down regulated, and the biomarker gene or gene product EDN1 is upregulated, and the biomarker genes or gene products of STC-1 and IL-8 is downregulated after administration of the inhibitor.

5. The inhibitors for use according to any of the claims 1 - 4, wherein the sample is a body fluid selected from the group consisting of blood, blood plasma, lymph, liquor or urine.

6. The inhibitors for use according to any of the claims 1 - 4, wherein the sample is tissue from endothelial cells.

7. The inhibitors for use according to any of the claims 1 - 6, wherein the up- and downregulation of the expression of said biomarker genes or gene products thereof refers to the expression of said biomarker genes or products before administration of the inhibitor.

8. The inhibitors for use according to any of the claims 1 - 7, for finding and optimizing of an effective dosing and/or an effective schedule.


SEQUENCE LISTING

<110> Merck Patent GmbH

<120> Biomarkers for Inhibitors with Anti-Angiogenic Activity

<130> P08/201-bz

<140> PCT/EP2009/008929
<141> 2008-12-23

<160> 120

<170> PatentIn version 3.3

<210> 1
<211> 214
<212> PRT
<213> Artificial

<220>
<223> mutated murine immunoglobulin chain

<220>
<221> CHAIN
<222> (1)..(214)
<223> DI17E6 variable and constant light chain.

<220>
<221> CHAIN
<222> (1)..(107)
<223> variable light chain

<220>
<221> BINDING
<222> (24)..(34)
<223> CDR1=Seq.ID.No.5

<220>
<221> BINDING
<222> (50)..(56)
<223> CDR2=Seq.ID.No.6

<220>
<221> BINDING
<222> (89)..(97)
<223> CDR3=Seq.ID.No.7

<220>
<221> CHAIN
<222> (108)..(214)
<223> constant light chain

<400> 1

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Ser Asn Tyr
            20                  25                  30

Leu Ala Trp Tyr Gln Gln Lys Pro Gly Lys Ala Pro Lys Leu Leu Ile
        35                  40                  45

Tyr Tyr Thr Ser Lys Ile His Ser Gly Val Pro Ser Arg Phe Ser Gly
    50                  55                  60

Ser Gly Ser Gly Thr Asp Tyr Thr Phe Thr Ile Ser Ser Leu Gln Pro
65                  70                  75                  80

Glu Asp Ile Ala Thr Tyr Tyr Cys Gln Gln Gly Asn Thr Phe Pro Tyr
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 2
<211> 447
<212> PRT
<213> Artificial

<220>
<223> mutated murine immunoglobulin chain

<220>
<221> CHAIN
<222> (1)..(447)
<223> DI17E6 variable and constant heavy chain
1D)

<220>
<221>  CHAIN
<222>  (1)..(118)
<223>  variable heavy chain

<220>
<221>  BINDING
<222>  (30)..(35)
<223>  CDR1=Seq.ID.No.8

<220>
<221>  BINDING
<222>  (50)..(66)
<223>  CDR2=Seq.ID.No.9

<220>
<221>  BINDING
<222>  (99)..(107)
<223>  CDR3=Seq.ID.No.10

<220>
<221>  CHAIN
<222>  (119)..(447)
<223>  constant heavy chain

<400>  2

Gln Val Gln Leu Gln Gln Ser Gly Gly Glu Leu Ala Lys Pro Gly Ala
1               5                   10                  15

Ser Val Lys Val Ser Cys Lys Ala Ser Gly Tyr Thr Phe Ser Ser Phe
            20                  25                  30

Trp Met His Trp Val Arg Gln Ala Pro Gly Gln Gly Leu Glu Trp Ile
        35                  40                  45

Gly Tyr Ile Asn Pro Arg Ser Gly Tyr Thr Glu Tyr Asn Glu Ile Phe
    50                  55                  60

Arg Asp Lys Ala Thr Met Thr Thr Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Ser Phe Leu Gly Arg Gly Ala Met Asp Tyr Trp Gly Gln Gly Thr
            100                 105                 110

Thr Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Asn Phe Gly Thr Gln Thr Tyr Thr Cys Asn Val Asp His Lys Pro Ser
        195                 200                 205

Asn Thr Lys Val Asp Lys Thr Val Glu Pro Lys Ser Ser Asp Lys Thr
    210                 215                 220

His Thr Cys Pro Pro Cys Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Ala Gln Ser Thr Phe Arg Val Val Ser
    290                 295                 300

Val Leu Thr Val Val His Gln Asp Trp Leu Asn Gly Lys Glu Tyr Lys
305                 310                 315                 320

Cys Lys Val Ser Asn Lys Gly Leu Pro Ala Pro Ile Glu Lys Thr Ile
                325                 330                 335

Ser Lys Thr Lys Gly Gln Pro Arg Glu Pro Gln Val Tyr Thr Leu Pro
            340                 345                 350

Pro Ser Arg Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Met Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly Lys
        435                 440                 445